(19) Europäisches Patentamt
European Patent Office
Office européen des brevets

(11) EP 3 548 544 B1

(12) **EUROPEAN PATENT SPECIFICATION**

(45) Date of publication and mention of the grant of the patent:
**21.07.2021 Bulletin 2021/29**

(21) Application number: **17804179.4**

(22) Date of filing: **24.11.2017**

(51) Int Cl.:
***A61K 8/44*** *(2006.01)*
***A61Q 1/02*** *(2006.01)*
***A61Q 5/00*** *(2006.01)*
***A61K 8/46*** *(2006.01)*
***A61Q 1/10*** *(2006.01)*
***A61K 8/86*** *(2006.01)*
***C08J 3/03*** *(2006.01)*
***A61K 8/81*** *(2006.01)*
***A61K 8/41*** *(2006.01)*
***A61Q 19/00*** *(2006.01)*

(86) International application number:
**PCT/EP2017/080391**

(87) International publication number:
**WO 2018/099827 (07.06.2018 Gazette 2018/23)**

(54) **AQUEOUS DISPERSION OF POLYALKENE SUPRAMOLECULAR POLYMER AND ITS USE IN COSMETICS**

WÄSSRIGE DISPERSION EINES SUPRAMOLEKULAREN POLYALKENPOLYMERS UND DEREN VERWENDUNG IN DER KOSMETIK

DISPERSION AQUEUSE DE POLYMÈRE SUPRAMOLÉCULAIRE DE POLYALCÈNE ET SON UTILISATION EN COSMÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2016 FR 1661698**

(43) Date of publication of application:
**09.10.2019 Bulletin 2019/41**

(73) Proprietor: **L'OREAL**
**75008 Paris (FR)**

(72) Inventors:
- **PECCHIA, Olivier**
**95500 Le Thillay (FR)**
- **CHODOROWSKI-KIMMES, Sandrine**
**93601 Aulnay-Sous-Bois (FR)**
- **ARNAUD-ROUX, Mireille**
**93601 Aulnay-Sous-Bois (FR)**

(74) Representative: **L'Oreal**
**Service D.I.P.I.**
**9, rue Pierre Dreyfus**
**92110 Clichy (FR)**

(56) References cited:
**EP-A1- 2 189 151**
**FR-A1- 2 938 758**
**US-A1- 2005 031 566**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 548 544 B1

## Description

The present invention relates to an aqueous dispersion of supramolecular polymer particles, a cosmetic composition comprising said aqueous dispersion and a cosmetic treatment process for keratinous materials comprising the application of such composition.

The supramolecular polymers are interesting in the cosmetic field, particularly for make-up products, skincare products or hair products, for their film-forming properties exhibiting good hold and non-transfer of the deposit during contact with clothing or a glass.

In application EP-A-2189151, make-up compositions are known containing a polymer of polycondensate type containing polyalkene polymer, diisocyanate and a compound with a supramolecular group like the group ureidopyrimidone. However, such a polymer is soluble in hydrocarbon organic solvents like isododecane and is therefore difficult to incorporate in aqueous cosmetic compositions such as shampoos, aqueous gels (for example care compositions in the form of serum) and aqueous emulsions. What is more, after spreading and evaporation of the organic solvent, this polymer in organic solution gives a film that exhibits a tacky appearance.

We have observed that such a polymer cannot be formulated in cosmetic compositions with high contents, for example greater than 30% by weight, because the mixture sets and the composition cannot be handled. Moreover, the film obtained with such a polymer does not exhibit optimal surface properties, particularly its properties of softness to the touch

Therefore a need exists to be able to formulate a supramolecular polymer particles with a polyalkene backbone in aqueous substrates and to obtain a film after application and drying that exhibits a non-tacky or low-tack appearance.

The inventors have discovered that an aqueous dispersion of the polyalkene supramolecular polymer particles may be obtained using a specific surfactant system as described hereinafter. Such an aqueous dispersion exhibits good stability properties, especially after storage at ambient temperature for 1 week or even for 1 month. What is more, the polymer film obtained after application of the aqueous dispersion to a substrate and evaporation of the water exhibits a non-tacky or low-tack appearance. The aqueous dispersion also allows the easy incorporation of the polyalkene supramolecular polymer particles in aqueous compositions without resorting to the use of organic solvents, especially in cosmetic compositions.

A subject of the invention is therefore an aqueous dispersion of a polyalkene supramolecular polymer particles with a specific surfactant system as described hereinafter.

Another subject of the invention is a composition, especially a cosmetic composition, comprising an aqueous dispersion as described previously.

Another subject of the invention is a cosmetic treatment process, especially a cosmetic process, for keratin materials, comprising the application to the keratin materials of an aqueous dispersion or of a composition, especially a cosmetic composition, containing the composition as defined previously.

The aqueous dispersion of polymer is presented in the form of a dispersion of polymer particles particles in water. The size of the polymer particles in dispersion in the aqueous phase may range from 5 nm to 600 nm, and preferably from 80 nm to 400 nm.

In the sense of the present invention, polyalkene supramolecular polymer is understood to mean a polymer including in its structure at least one polyalkene portion and at least one portion including at least one group that can form at least three hydrogen bonds, preferably four hydrogen bonds.

The supramolecular polymers of the invention may especially be from the condensation of at least one polyalkene polymer (A) functionalized by at least one reactive group, with at least one functionalized graft (B) by at least one reactive group that can react with the reactive group or groups of the functionalized polyalkene polymer, said graft bearing at least one group that can form at least three hydrogen bonds, preferably four hydrogen bonds.

Preferably, the functionalized polyalkene polymer (A) has formula A1:

HX-P-X'H

- where XH and X'H are reactive groups, with X and X', which may be identical or different, chosen from O, S, NH and $NR_a$, $R_a$ representing a linear or branched $C_1$-$C_6$ alkyl group;
- P represents a homo- or copolymer that can be obtained by polymerization of one or more linear, cyclic and/or branched, mono- or polyunsaturated $C_2$-$C_5$ alkenes.

P preferably represents a polyethylene, a polybutylene, a polybutadiene, a hydrogenated polybutadiene, a polyisoprene, a poly(1,3-pentadiene), a polyisobutylene, and their copolymers such as poly(ethylenebutylene), preferably a hydrogenated polybutadiene.

Poly(ethylene/butylenes) are copolymers of 1-butene and of ethylene. They may be represented schematically

by the following sequence of units: [$-CH_2-CH_2-$] and [$-CH_2CH(CH_2-CH_3)-$]

The polybutadienes may be 1,4-polybutadienes or 1,2-polybutadienes, which may be represented schematically, respectively, by the following sequences of units:

[$-CH_2-CH=CH-CH_2-$] (1,4-polybutadienes)
[$-CH_2-CH(CH=CH_2)-$] (1,2-polybutadienes)

Preferably, they are 1,2-polybutadienes.

Polyisoprenes may be represented schematically by the following sequences of units:

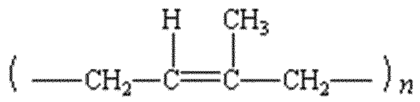 and 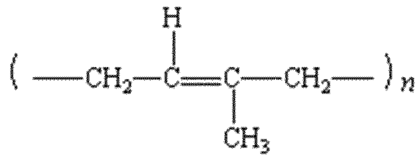

A mixture of above units may obviously also be used, so as to form copolymers.

The functionalized polyalkene polymer is preferably functionalized at the end of the chain. They are then referred to as telechelic polymers.

Preferably, the functionalized polyalkene polymers have a number-average molecular mass (Mn) of greater than or equal to 1000, especially between 1000 and 5000, or even between 1500 and 3500.

The functionalized polyalkene polymers may be totally hydrogenated to avoid the risks of crosslinking.

Preferably, for the polyalkene polymer HX-P-X'H, X and X' are identical. Particularly X = X' = O.

Among the preferred HX-P-X'H polyalkene polymers, mention may be made of polydienes, which are preferably hydrogenated, containing hydroxyl functions, preferably hydroxyl end groups, and polyolefins containing hydroxyl end groups.

The polydienes containing hydroxyl end groups are especially defined, for example, in FR 2 782 723. They may be chosen from polybutadiene, polyisoprene and poly(1,3-pentadiene) homopolymers and copolymers. Preferably, they have a number-average molecular mass (Mn) of less than 7000, preferably between 1000 and 5000; and present functionality at the hydroxyl ends of 1.8 to 3, and preferably in the region of 2. Mention will be made in particular of the hydroxylated polybutadienes sold by the company Cray Valley under the brand names Poly BD R-45HT and Poly BD R-20 LM, which will preferably be used hydrogenated; and also hydrogenated dihydroxylated (1,2-polybutadienes), such as GI3000 of Mn = 3100, GI2000 (Mn = 2100) and GI1000 (Mn = 1500) sold by the company Nisso.

Among the polyolefins with hydroxyl end groups, mention may be made preferably of polyolefins, homopolymers or copolymers with $\alpha,\omega$-hydroxyl end groups, such as polyisobutylenes with $\alpha,\omega$-hydroxyl end groups; and the copolymers having formula:

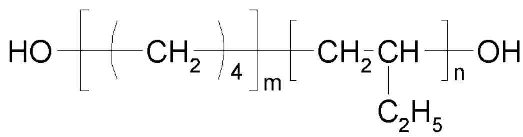

especially those sold by Mitsubishi under the brand name Polytail.

The supramolecular polymers of the present invention have in their structure at least one graft bearing at least one group that can form at least three hydrogen bonds, preferably at least four hydrogen bonds.

The groups that can form at least three hydrogen bonds may comprise for example at least three functional groups, preferably at least four functional groups, chosen from:

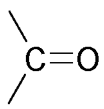 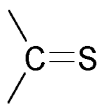 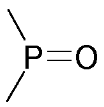 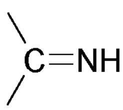 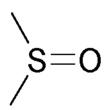

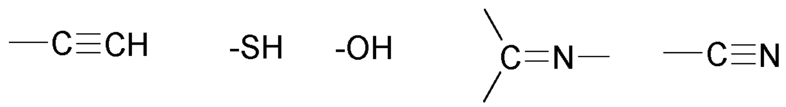

-NH2

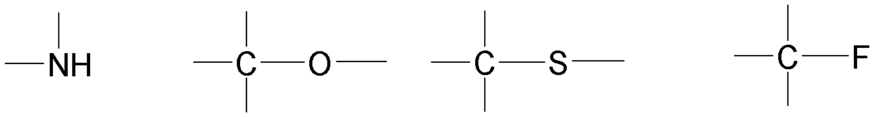

These functional groups may be classified into two categories:

- hydrogen bond donor functional groups such as the groups:

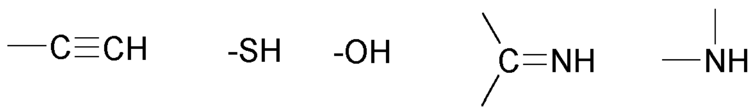

  -NH2

- and hydrogen bond acceptor functional groups such as the groups:

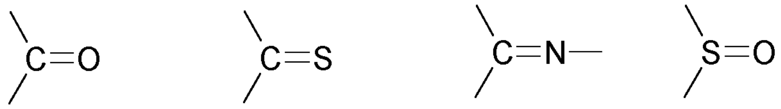

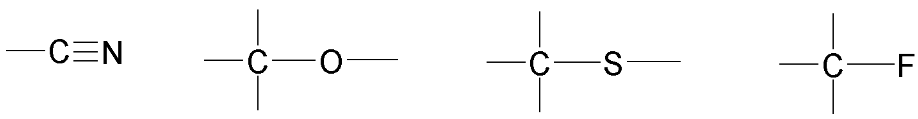

The groups that can form at least three hydrogen bonds form a basic structural element including at least three groups, preferably at least four groups and more preferably four functional groups capable of establishing hydrogen bonds. The basic structural elements capable of establishing three or four hydrogen bonds may be represented schematically in the following manner:

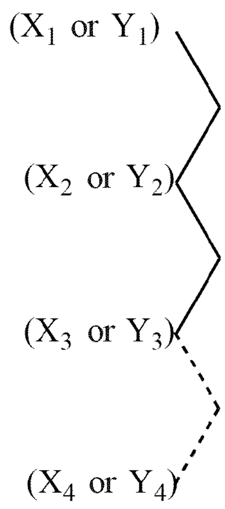


where $X_i$ (i is a natural integer) is a hydrogen bond acceptor functional group and $Y_i$ is a hydrogen bond donor functional

group.

Thus, each structural element should be capable of establishing hydrogen bonds with one or more partner structural elements, which are identical (i.e. self-complementary) or different, such that each pairing of two partner structural elements takes place by the formation of at least three hydrogen bonds, preferably at least four hydrogen bonds and more preferably four hydrogen bonds.

A proton acceptor X will pair with a proton donor Y.

Several possibilities are thus offered, for example pairing of:

XXXX with YYYY;
XXXY with YYYX;
XXYX with YYXY;
XYYX with YXXY;
XXYY with YYXX self-complementary or otherwise;
XYXY with YXYX self-complementary or otherwise.

Preferably, the groups may establish four hydrogen bonds with an identical (or self-complementary) partner group among which are two donor bonds (for example NH) and two acceptor bonds (for example CO and -C=N-).

Preferably, the groups that can form at least three hydrogen bonds include 5- or 6-membered rings (aromatic rings or unsaturated heterocycles) very often constituted of C and/or N atoms and with conjugated double bonds to stabilize and direct the hydrogen interactions.

Even more preferably, the groups that can form at least three hydrogen bonds are part of 6-membered rings comprising C and/or N atoms and with conjugated double bonds to stabilize and direct the hydrogen interactions.

According to a specific embodiment of the invention, the groups that can form at least three or four hydrogen bonds are chosen from the following families, it being understood that all the tautomeric forms are included:

- (i) aminopyrimidones having formula:

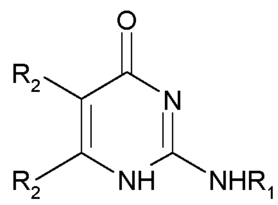

- (ii) ureidopyrimidones having formula:

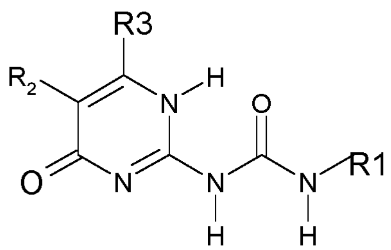

(iia) imidazolidones having formula:

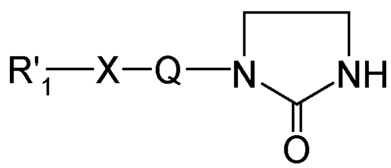

in which:

Q denotes a divalent hydrocarbon substituent having from 1 to 10 carbon atoms, optionally interrupted by a -COO-

-OCO-, -CO-, -O-, -S-, -NH-, -CONH- group;
X denotes a divalent nucleophilic group chosen from -NH-,-$NR_b$-, -O-, -S-, -OOC-; $R_b$ denotes a $C_1$-$C_4$ alkyl group;
R'1 denotes a single bond constituting the connection point on the rest of the graft.

- (iii) acylaminopyridines and especially:
- monoacylaminopyridines having structure:

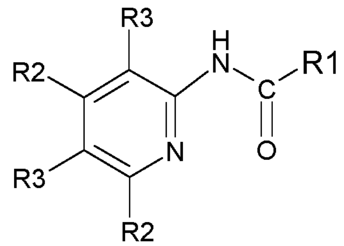

- di(acylamino)pyridines and more particularly 2,6-di(acylamino)pyridines having structure:

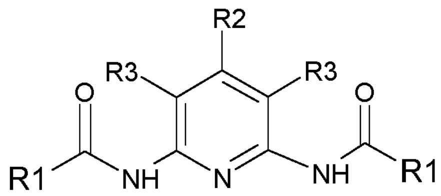

- (iv) aminopyrimidines, and especially:
- aminopyrimidine compounds:

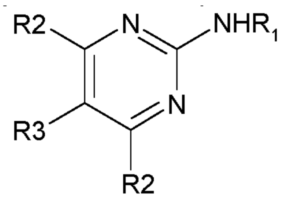 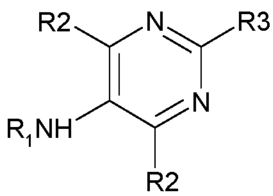 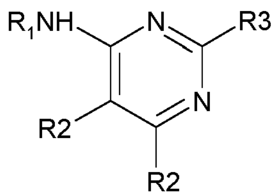

- diaminopyrimidine compounds having structure:

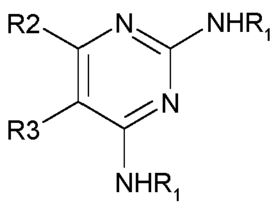 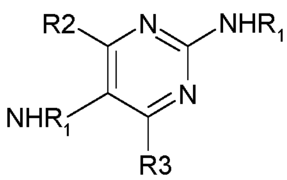 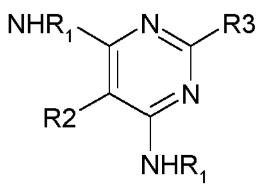 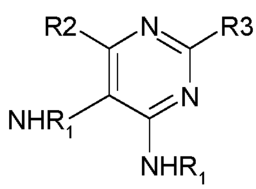

- triaminopyrimidine compounds;
- (v) ureidotriazines, and especially mono-, di- and tri-ureidotriazines, and in particular ureidoaminotriazines having structure:

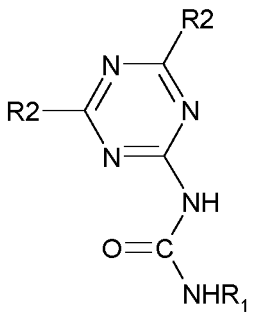

- (vi) (acylamino)triazines and especially mono-, di- and tri-acylamino triazines, optionally amino (mono-, di- or triamino), in particular:
- di(acylamino)triazines having structure:

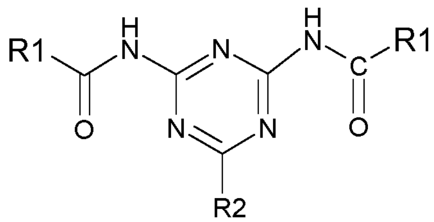

- acylamino, amino-triazines, (mono- or diacylamino, and mono- or diamino) and especially compounds having structure:

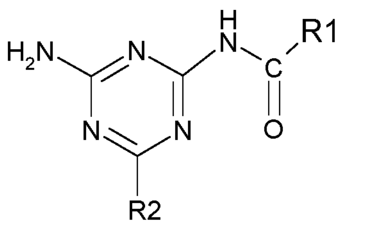

- acylaminotriazines having structure:

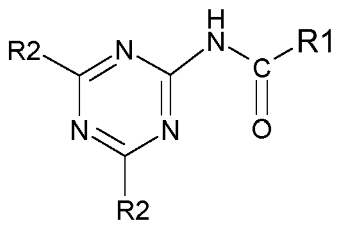

- tri-acylaminotriazines;
- (vii) aminotriazines, and especially:
- monoaminotriazines;
- 2,6-diamino-s-triazines having structure:

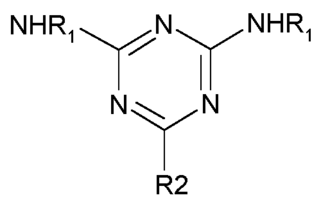

- triamino-s-triazine compounds having structure:

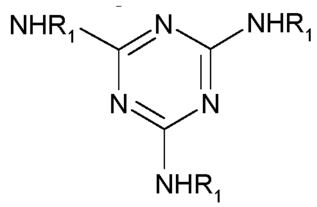

- (viii) acylaminotriazoles having structure:

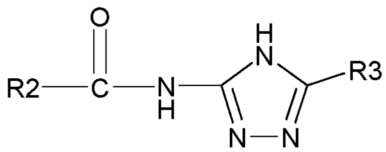

- (ix) compounds of the urazoylbenzoic acid family having structure:

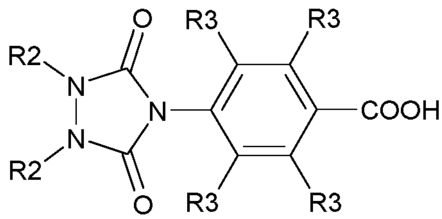

- (x) phthalhydrazides having structure:

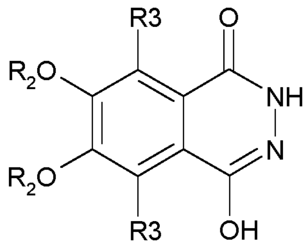

- (xi) uracils having structure:

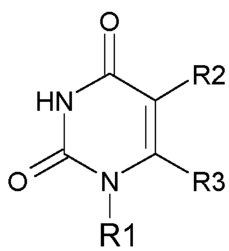

- (xii) thymines having structure:

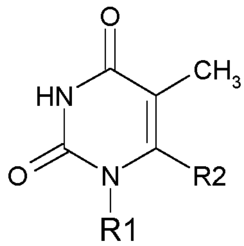

- (xiii) succinimides having structure:

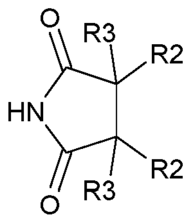

- (xiv) glutarimides having structure:

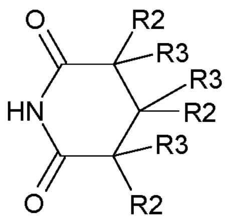

- (xv) compounds of the cyanuric acid family having structure:

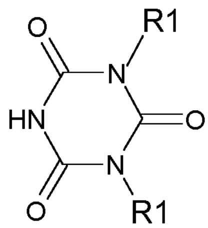

- (xvi) maleimides:

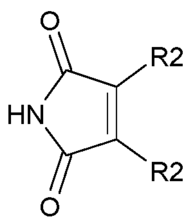

- (xvii) compounds of the barbituric acid family, having structure:

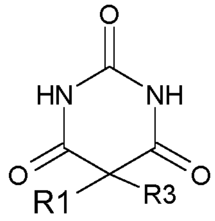

- (xviii) compounds having structure:

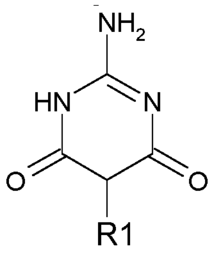

et

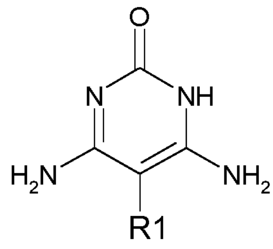

- (xix) compounds of the trimellitic acid family, having formula:

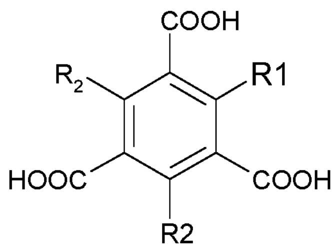

- (xx) ureidopyridines, especially mono- or di-ureidopyridines, and in particular those having formula:

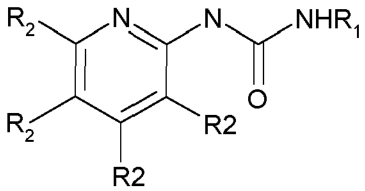

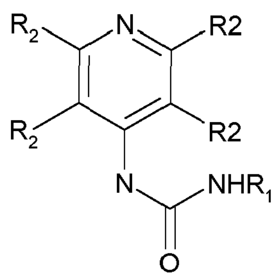

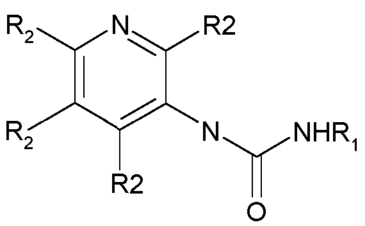

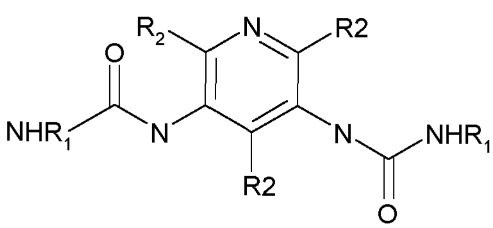

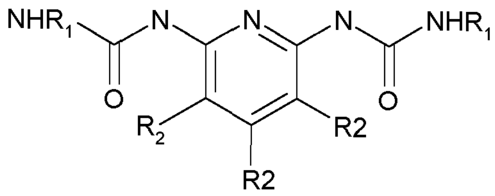

- (xxi) carbamoylpyridines having formula:

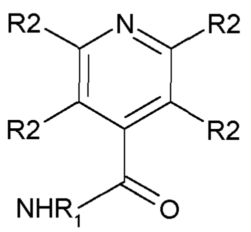

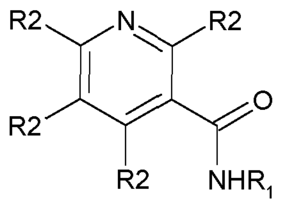

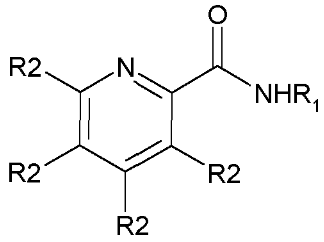

- (xxii) adenines having formula:

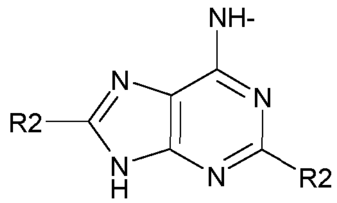

- (xxiii) guanines having formula:

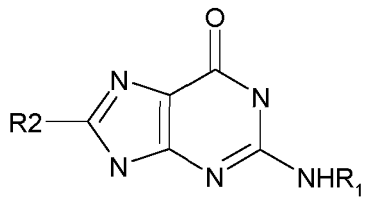

- (xxiv) cytidines having formula:

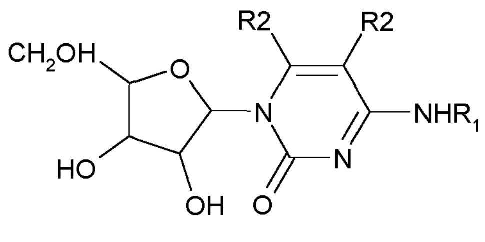

In all of these formulae, the substituents have the following meanings:

- (a) the substituents $R_1$, which may be identical or different, represent a single bond, a hydrogen atom, a halogen atom and/or a linear, branched or cyclic, $C_1$-$C_{6000}$, saturated or unsaturated, optionally aromatic monovalent carbon-based group (especially alkyl), which may contain one or more heteroatoms such as O, S, N, P, Cl, Br, F; or a combination of these meanings.

The substituent $R_1$ may especially be a $C_4$-$C_{12}$ cycloalkyl group, a linear or branched $C_1$-$C_{30}$ alkyl group or a

$C_4$-$C_{12}$ aryl group, optionally substituted with an amino, ester and/or hydroxyl function.

The substituent $R_1$ may also be a group: $C_4H_9$, phenyl, 1,4-nitrophenylene, 1,2-ethylene, 1,6-hexylene; 1,4-butylene, 1,6-(2,4,4-trimethylhexylene), 1,4-(4-methylpentylene), 1,5-(5-methylhexylene), 1,6-(6-methylheptylene), 1,5-(2,2,5-trimethylhexylene), 1,7-(3,7-dimethyloctylene), -isophorone-, 4,4'-methylene bis(cyclohexylene), tolylene, 2-methyl-1,3-phenylene, 4-methyl-1,3-phenylene, 4,4-biphenylenemethylene,
and preferably: -isophorone-, $-(CH_2)_2-$, $-(CH_2)_6-$, $CH_2CH(CH_3)-CH_2-C(CH_3)_2-CH_2-CH_2$, 4,4'-methylene biscyclohexylene, 2-methyl-1,3-phenylene.

Even more preferably, $R_1$ is a single bond.

- (b) the substituents $R_2$, which may be identical or different within the same formula, represent a single bond, a hydrogen atom, a halogen atom (-Br, -Cl, -F), a -OH, $-N(R)_2$ substituent (with R being H or a linear and branched $C_1$-$C_{12}$ and preferably a $C_1$-$C_4$ alkyl substituent, and better still a methyl or ethyl substituent); or a linear, branched or cyclic, $C_1$-$C_{6000}$, saturated or unsaturated, optionally aromatic monovalent hydrocarbon-based group, which may contain one or more heteroatoms such as O, S, N, P and F; or a combination of these meanings.

The substituents $R_2$ may especially be H, CN, $NH_2$ or:

- a $C_1$-$C_{30}$ alkyl group;
- a $C_4$-$C_{12}$ cycloalkyl group;
- a $C_4$-$C_{12}$ aryl group;
- a ($C_4$-$C_{12}$)aryl($C_1$-$C_{30}$)alkyl group;
- a $C_1$-$C_4$ alkoxy group;
- an arylalkoxy group, in particular an aryl($C_1$-$C_4$)alkoxy group;
- a C4-C12 heterocycle;
- a thioalkoxy group;
- a sulfoxy group;

or mixtures thereof, these groups optionally being substituted with an amino, ester and/or hydroxyl function.

Preferably, $R_2$ represents H, $CH_3$, $C_{13}H_{27}$, $C_7H_{15}$ or phenyl.

- (c) the substituents $R_3$, which may be identical or different within the same formula, represent a hydrogen atom or a linear, branched or cyclic, $C_1$-$C_{6000}$, saturated or unsaturated, optionally aromatic monovalent hydrocarbon-based group, which may contain one or more heteroatoms such as O, S, N, P or F; or a combination of these meanings.

The substituent $R_3$ may especially be a $C_4$-$C_{12}$ cycloalkyl group, a linear or branched $C_1$-$C_{30}$ alkyl group or a $C_4$-$C_{12}$ aryl group, optionally substituted with an amino, ester and/or hydroxyl function. Preferably, substituent $R_3$ represents a methyl substituent. In all of these formulae, it is clearly understood that at least one, especially one or two, of the groups $R_1$ and/or $R_2$ is a single bond constituting the point of attachment of the group that can form at least three hydrogen bonds to the rest of the graft.

Preferably, said point of attachment is borne by $R_1$ and/or $R_2$, and preferably it is borne by $R_1$.

The groups that can form at least three hydrogen bonds are chosen especially from:

(a) groups that can form at least three complementary and identical hydrogen bonds, i.e. self-complementary, and especially:

- aminopyrimidones, ureidopyrimidones, imidazolidones,
- compounds of the trimellitic acid family, or of the urazoylbenzoic acid family,
- acylaminopyridines, ureidopyridines, carbamoylpyridines,
- acylaminotriazines, ureidotriazines, and especially ureidoaminotriazines, diaminotriazines,
- acylaminotriazoles,
- phthalhydrazides,
- compounds having formula:

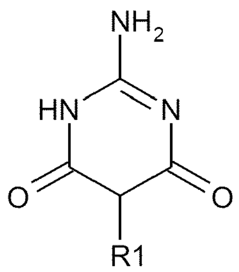
and
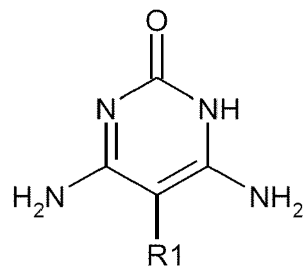

in which $R_1$ is a hydrogen atom or a linear, branched or cyclic, $C_1$-$C_{6000}$, saturated or unsaturated, optionally aromatic monovalent hydrocarbon-based group, which may contain one or more heteroatoms such as O, S, N, P and F,

(b) groups that can form at least three complementary but different hydrogen bonds and especially:

- adenine, which is complementary to guanine,
- cytidine, which is complementary to thymine,
- triamino-s-triazine, which is complementary to uracil or succinimide or glutarimide or cyanuric acid or thymine or maleimide or (di)aminopyrimidine or barbituric acid,
- acylamino-amino-s-triazine, which is complementary to uracile or succinimide or glutarimide or cyanuric acid or thymine or maleimide or (di)aminopyrimidine or barbituric acid.

In a preferred manner, the groups that can form at least three hydrogen bonds are chosen from groups that can establish at least three hydrogen bonds with themselves (self-complementary), especially at least four hydrogen bonds with themselves. Among these groups, mention may be made in particular of:

- ureidopyrimidones; imidazolidones;
- ureidopyridines, carbamoylpyridines;
- acylamino-s-triazines and especially acyl-diamino-s-triazines;
- ureidotriazines;
- phthalhydrazides;
- compounds having formula:

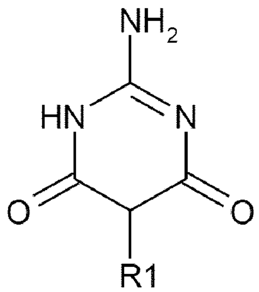
and
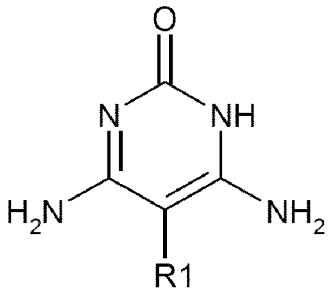

in which the substituents $R_1$, $R_2$ and $R_3$ have the meanings given above, in particular the meanings given as a preference.

Better still, as preferred examples of groups that can form at least three hydrogen bonds, mention may be made of groups derived from ureidopyrimidones and imidazolidines, preferably groups derived from ureidopyrimidones and in particular from 2-ureidopyrimidone or 6-methyl-2-ureidopyrimidone.

The rest of the functionalized graft is constituted of a linker L bearing at least one reactive group (W) that can react with the reactive group or groups of the functionalized poly(alkene).

This reactive group (W) may be for example a carboxyl group or an isocyanate group. Preferably, it is a -N=C=O or -N=C=S group, and even more preferably, a - N=C=O group (isocyanate).

Preferably, linker L is a divalent saturated or unsaturated C2-C20, preferably C6-C20 hydrocarbon-based substituent. Preferably, linker L is a divalent saturated or unsaturated cyclic $C_6$-$C_{20}$, preferably $C_6$-$C_{12}$ hydrocarbon-based substituent. A divalent cyclic hydrocarbon-based substituent is understood to mean a substituent containing in its structure at least one cyclic portion.

Linker L may be a divalent substituent chosen from phenylene, 1,2-ethylene, 1,6-hexylene, 1,4-butylene,

1,6-(2,4,4-trimethylhexylene), 1,4-(4-methylpentylene), 1,5-(5-methylhexylene), 1,6-(6-methylheptylene), 1,5-(2,2,5-trimethylhexylene), 1,7-(3,7-dimethyloctylene), -isophorone-, 4,4'-methylene bis(cyclohexylene), tolylene, 2-methyl-1,3-phenylene, 4-methyl-1,3-phenylene, 4,4-biphenylenemethylene.

L preferably represents a divalent group: phenylene, -isophorone-, 4,4'-methylene biscyclohexylene, tolylene, 2-methyl-1,3-phenylene, 4-methyl-1,3-phenylene, 4,4-biphenylenemethylene,
and preferably -isophorone-, 4,4'-methylene biscyclohexylene, 2-methyl-1,3-phenylene.

Preferably, L is the divalent group -isophorone-.

Divalent isophorone group is understood to mean the following group:

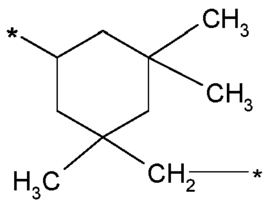

* representing the attachment points for the group in the polymer backbone.

In a particularly preferred version of the invention, the functionalized grafts (B) have formula (B1):

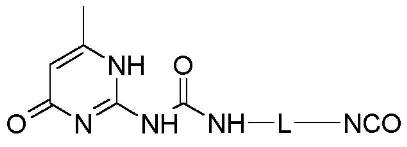
(B1)

or have the formula (B2):

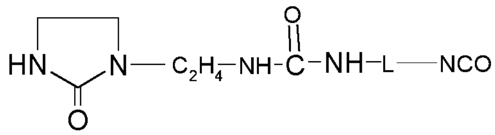
(B2)

Where L has the same meaning as above.

Even more preferably, the polyalkene supramolecular polymer has formula (C1):

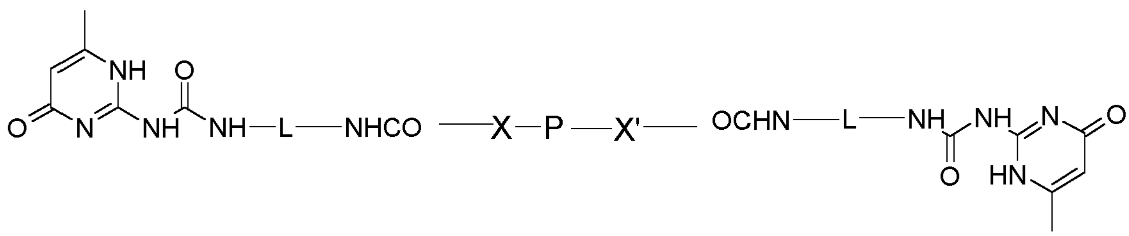

(C1)

P, X, X', L having the meanings indicated previously.
or having formula (C2):

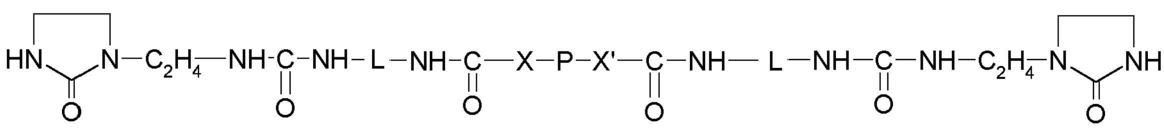

(C2)

P, X, X', L having the meanings indicated previously.

Preferably, X= X'= O.

Preferably, in formulas (C1), (C2), X and X' denote an oxygen atom.

The polyalkene supramolecular polymer or polymers of the invention may also be obtained from a polymer (A1) including a polyalkene portion, said polymer being functionalized by at least one reactive group (B1), which reacts by condensation with at least one molecule (A2) including at least one reactive group (B2), said molecule being such that after reaction of groups (B1) and (B2) an entity forms that can form at least three hydrogen bonds, preferably at least four hydrogen bonds.

Preferably, these entities are structures (i) to (xxiv) as defined previously, with $R_1$ denoting a single bond.

Polymer (A1) may especially result from the action on a functionalized polyalkene polymer having formula A as defined before, of compounds (A2) including two reactive groups (B2) and (B'2) that can react with the functionalized groups of the polyalkene.

These reactive groups may for example be carboxyl groups or isocyanate groups. Preferably, it is -N=C=O or -N=C=S groups, and even more preferably an -N=C=O group (isocyanate).

Preferably, B2 groups are identical to B'2 groups.

Preferably, the compounds (A2) have the following structure (C'):

B'2-L-B'2 (C')

where linker L has the same meanings as those defined previously.

In a particularly preferred version of the invention, the polymers A1 have formula (C'1):

CON-L-NCO-X-P-X'-CON-L-NCO (C'1)

in which L, X, X' and P have the same meanings as those described previously. Preferably, molecule (A2) is 6-methylisocytosine having formula:

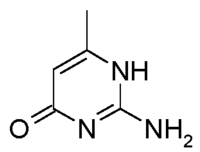

or 2-aminoethylimidazolidine (UDETA) having formula:

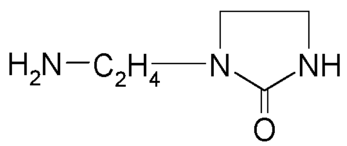

In practice, the polyalkene supramolecular polymer may be prepared according to a preparation process consisting in:

- heating polymer (A1) including at least one reactive group, especially two reactive groups, particularly two OH groups, to a temperature that can be inclusively between 60 °C and 140 °C, ensuring in advance that the polymer does not include residual water.
- adding at least one, preferably only one, functionalized graft by at least one reactive group that can react with the reactive group or groups on the functionalized polyalkene polymer;
- stirring the mixture under a controlled atmosphere at a temperature of the order of 90-130°C; for 1 to 24 hours;
- monitoring reaction progress for example by assaying the reactive groups borne by the polymer (for example by finding the hydroxyl indices if the polymer bears hydroxyl groups) and/or by monitoring the disappearance of the reactive groups borne by the graft or grafts (for example by monitoring by infrared spectroscopy the disappearance of the characteristic isocyanate band between 2500 and 2800 $cm^{-1}$ so as to stop the reaction when the peak completely disappears)
- letting the finished product return to ambient temperature;
- optionally adding a compound G-XH and/or G-X'H to ensure the complete disappearance of the reactive groups borne by the graft; G denotes a hydrogen atom or a C1-C12 linear or branched alkyl substituent; particularly, X, X'

being as defined previously, GXH denotes the ethanol if the graft bears isocyanate functions;

- filtering the mixture if necessary.

The reaction may be performed in the presence of a solvent or a mixture of solvents, in particular chosen from methyltetrahydrofuran, tetrahydrofuran, toluene or butyl acetate, or propylene carbonate.

It is also possible to add a conventional catalyst to achieve the condensation between the functionalized polymer and the functionalized graft.

The resulting compound may finally be washed and dried, or even purified, according to the general knowledge of those skilled in the art.

According to a preferred embodiment, the polyalkene supramolecular polymer may be prepared according to a preparation process consisting in:

- heating the polyalkene polymer that is di-functionalized by hydroxyl functions (preferably functionalized at the ends of the chain) (polymer A1) at a temperature that can be comprised between 60 °C and 140 °C, ensuring previously that it does not include residual water;
- adding a functionalized isocyanate, preferably diisocyanate, graft.
- stirring the mixture under a controlled atmosphere at a temperature of the order of 90-130°C; for 1 to 24 hours;
- monitoring reaction progress for example by assaying the reactive groups borne by the polymer (for example by finding the hydroxyl indices if the polymer bears hydroxyl groups) and/or by monitoring the disappearance of the reactive groups borne by the graft or grafts (for example by monitoring by infrared spectroscopy the disappearance of the characteristic isocyanate band between 2500 and 2800 $cm^{-1}$ so as to stop the reaction when the peak completely disappears)
- letting the finished product return to ambient temperature;
- optionally a compound G-XH and/or G-X'H to ensure the complete disappearance of the reactive groups borne by the graft; G denotes a hydrogen atom or a C1-C12 linear or branched alkyl substituent; X and X' being as defined previously (preferably denote O); in particular, GXH and GX'H denote the ethanol if the graft bears isocyanate functions;
- filtering the mixture if necessary.

The reaction may be performed in the presence of a solvent or a mixture of solvents, in particular chosen from methyltetrahydrofuran, tetrahydrofuran, toluene or butyl acetate, or propylene carbonate.

It is also possible to add a conventional catalyst to achieve the condensation between the functionalized polymer and the functionalized graft. As an example, mention may be made of dibutyltin dilaurate if we wish to form a urethane bond between an hydroxyl-functionalized polymer and a functionalized isoscyanate graft.

The compound may finally be washed and dried, or even purified, according to the general knowledge of those skilled in the art.

According to another embodiment, the supramolecular polymer may be prepared according to a process comprising the following steps:

(i) Functionalization of the dihydroxylated polyalkene polymer P, previously dried, by a diisocyanate according to the reaction scheme:

$$\text{OH-P-OH (x eq) + NCO-L-NCO (y eq) (D)} \rightarrow \text{OCN-L-NH-(O)CO-P-OC(O)-NH-L-NCO}$$

preferably in quantities such that the polyalkene polymer/diisocyanate D molar ratio (ratio x/y) ranges from 0.30 to 0.70, better still from 0.35 to 0.65, preferably from 0.4 to 0.6, more preferably from 0.45 to 0.55.

This first step may be made in the presence of solvent, at a temperature of between 20 °C and 100 °C.

This first step may be followed by a period of stirring, in a controlled atmosphere for a period ranging from 1 hour to 24 hours. The mixture may optionally be heated. The degree of progress of this first step may be monitored by assaying the hydroxyl functions; then

(ii) reaction of the pre-polymer obtained in step (i) with 6-methylisocytosine or 2-aminoethylimidazolidine:

OCN-L-NH-(O)CO-P-OC(O)-NH-L-NCO +

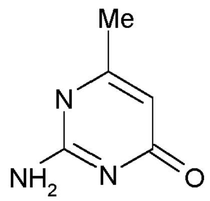

z eq

t eq

or

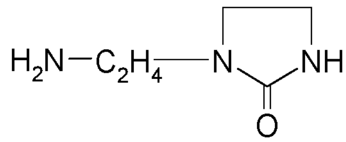

t eq

preferably in a quantity such that the diisocyanate D/6-methylisocytosine or 2-aminoethylimidazolidine molar ratio (ratio y/t) ranges from 0.80 to 1.20.

This second step may optionally be performed in the presence of a cosolvent such as toluene, butyl acetate or propylene carbonate. The reaction mixture may be heated to between 80°C and 140°C for a time ranging between 1 and 24 hours. The presence of a catalyst, such as for example dibutyltin dilaurate, may promote the production of the desired final product.

The reaction may be monitored by infrared spectroscopy, by monitoring the disappearance of the peak characteristic of isocyanate between 2200 and 2300 $cm^{-1}$.

At the end of the reaction, ethanol may be added to the reaction medium in order to neutralize any residual isocyanate functions. The reaction mixture may be optionally filtered. If necessary, the polymer may be directly stripped in a cosmetic solvent. As polyalkene supramolecular polymer, those described in application FR-A-2938760 can be used, particularly the polymer of example 3.

**Surfactant system of the aqueous dispersion:**

The aqueous dispersion of polyalkene supramolecular polymer particles according to the invention also comprises a surfactant system that can maintain said polymer in dispersion in water stably for at least one week at ambient temperature (23 °C).

The surfactant system is chosen from:

1) at least one anionic surfactant optionally combined with at least one non-ionic surfactant, with the exclusion of the surfactant system containing only dodecyl sulfate and/or an alkali metal salt of dodecyl sulfate;

2) at least one cationic surfactant, optionally combined with at least one non-ionic surfactant;

3) at least one non-ionic surfactant having an HLB greater than 10 or a mixture of non-ionic surfactants, without being combined with an ionic surfactant, said mixture having an HLB greater than 10.

According to a first embodiment of the invention, the surfactant system is one or more anionic surfactant(s).

This first embodiment excludes dodecyl sulfate and/or an alkali metal salt of dodecyl sulfate used as the only surfactant.

The term "anionic surfactant" means a surfactant comprising, as ionic or ionizable groups, only anionic groups.

In the present description, a species is termed "anionic" when it bears at least one permanent negative charge or when it can be ionized into a negatively charged species, under the conditions of use of the composition of the invention (for example the medium or the pH) and not comprising any cationic charge.

The anionic surfactants may be sulfate, sulfonate and/or carboxylic (or carboxylate) surfactants. Needless to say, a mixture of these surfactants may be used.

It is understood in the present description that:

- the carboxylate anionic surfactants comprise at least one carboxylic or carboxylate function (-COOH or $-COO^-$) and may optionally also comprise one or more sulfate and/or sulfonate functions;
- the sulfonate anionic surfactants comprise at least one sulfonate function ($-SO_3H$ or $-SO_3^-$) and may optionally also comprise one or more sulfate functions, but do not comprise any carboxylate functions; and
- the sulfate anionic surfactants comprise at least one sulfate function but do not comprise any carboxylate or sulfonate functions.

The carboxylic anionic surfactants therefore include at least one carboxylic or carboxylate function (-COOH or $-COO^-$).

They may be chosen from the following compounds: acylglycinates, acyllactylates, acylsarcosinates, acylglutamates; alkyl-D-galactosideuronic acids, alkyl ether carboxylic acids, alkyl(C6-30 aryl) ether carboxylic acids, alkylamido ether carboxylic acids; and also the salts of these compounds;
the alkyl and/or acyl groups of these compounds including from 6 to 30 carbon atoms, especially from 10 to 22, better still from 10 to 16 carbon atoms; said alkyl groups being linear or branched, where the aryl group preferably denotes a phenyl or benzyl group;
these compounds may be polyoxyalkylenated, especially polyoxyethylenated, and then preferably including from 1 to 50 ethylene oxide units and better still from 2 to 10 ethylene oxide units.

Use may also be made of the C6-C24 alkyl monoesters of polyglycoside-polycarboxylic acids, such as C6-C24 alkyl polyglycoside-citrates, C6-C24 alkyl polyglycoside-tartrates and C6-C24 alkyl polyglycoside-sulfosuccinates, and salts thereof.

The salts of these compounds may be alkali metal (particularly sodium) or alkaline earth, ammonium, or aminoalcohol salts.

Preferably, the carboxylic anionic surfactants are chosen from acylsarcosinates or acylglycinates whose acyl group includes 10 to 22 carbon atoms and more particularly a linear acyl group including from 10 to 16 carbon atoms, particularly the sodium salt of N-lauroyl sarcosine and sodium N-cocoyl glycinate. Preferably, the acylsarcosinates cited previously are used and more particularly the sodium salt of N-lauroyl sarcosine.

The sulfonate anionic surfactants include at least one sulfonate function ($-SO_3H$ or $-SO_3^-$).

They may be chosen from the following compounds: alkylsulfonates, alkylamidesulfonates, alkylarylsulfonates, $\alpha$-olefinsulfonates, paraffin sulfonates, alkylsulfosuccinates, alkyl ether sulfosuccinates, alkylamidesulfosuccinates, alkylsulfoacetates, N-acyltaurates, acylisethionates; alkylsulfolaurates; and also the salts of these compounds;
the alkyl groups of these compounds comprising from 6 to 30 carbon atoms, in particular from 12 to 28, better still from 14 to 24 or even from 16 to 22 carbon atoms; where the aryl group preferably denotes a phenyl or benzyl group;
where these compounds may be polyoxyalkylenated, in particular polyoxyethylenated, and then preferably comprising from 1 to 50 ethylene oxide units and better still from 2 to 25 ethylene oxide units, and preferably from 2 to 10.

The salts of these compounds may be alkali metal (particularly sodium) or alkaline earth, ammonium, or aminoalcohol salts.

The sulfate anionic surfactants that may be used comprise at least one sulfate function ($-OSO_3H$ or $-OSO_3^-$).

They may be chosen from the following compounds: alkyl sulfates, alkyl ether sulfates, alkylamido ether sulfates, alkylaryl polyether sulfates, monoglyceride sulfates; and the salts of these compounds;
the alkyl groups of these compounds including from 6 to 30 carbon atoms, in particular from 12 to 28, better still from 14 to 24 or even from 16 to 22 carbon atoms; where the aryl group preferably denotes a phenyl or benzyl group;
these compounds may be polyoxyalkylenated, especially polyoxyethylenated, and then preferably including from 1 to 50 ethylene oxide units and better still from 2 to 25 ethylene oxide units, and preferably from 2 to 10.

The salts of these compounds may be alkali metal (particularly sodium) or alkaline earth, ammonium, or aminoalcohol salts.

Preferentially, the sulfate anionic surfactants are chosen from:

- alkyl sulfates especially C6-C24, even C8-C20, such as dodecyl sulfate (not used as the only surfactant but always combined with a non-ionic surfactant) or decyl sulfate
- alkyl ether sulfates, especially C6-C24, even C12-C22, preferably comprising from 2 to 25 ethylene oxide units, more preferably 2 to 10 ethylene oxide units, such as lauryl ether sulfate comprising from 2 to 25 ethylene oxide units particularly in the form of alkali metal or alkaline earth metal, ammonium, or aminoalcohol salts, more particularly in the form of alkali metal salts such as sodium salts.

Preferably, the anionic surfactant is chosen from carboxylate surfactants and sulfate surfactants described previously.

When the anionic surfactant is in salt form, said salt may be chosen from alkali metal salts, such as the sodium or potassium salt, ammonium salts, amine salts and in particular amino alcohol salts, and alkaline-earth metal salts, such as the magnesium salt.

Examples of amino alcohol salts that may be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.

Alkali metal or alkaline earth metal salts and in particular sodium or magnesium salts, preferably sodium, are preferably used.

The anionic surfactant is preferably chosen from sodium N-lauroyl sarcosinate, sodium laureth sulfate and sodium decyl sulfate.

The surfactant system may also comprise a non-ionic surfactant as described hereinafter in combination with the anionic surfactant as described previously.

Advantageously, the non-ionic surfactant combined may be chosen from:

C8-C30 poloxyethylenated fatty alcohols, especially C12-C18 fatty alcohols, particularly polyoxyethylenated lauryl, cetyl, myristyl, stearic alcohols having from 2 to 30 moles of ethylene oxide;

polyoxyethylenated C8-C30 fatty acid esters (preferably C12-C18) of sorbitan especially polyoxyethylenated esters of C12-C18 fatty acids, in particular lauric, myristic, cetylic or stearic acids, of sorbitan especially containing from 2 to 30 mol of ethylene oxide;

polyglycerolated C8-C30 fatty acid esters, especially polyglycerolated esters of C12-C18 fatty acids, in particular lauric, myristic, palmitic, stearic or isostearic acid, especially containing from 2 to 16 mol of glycerol.

The non-ionic surfactant combined is preferably chosen from:
4 EO or 23 EO (Laureth-23 or Laureth-4) polyoxyethylenated lauryl alcohols polyoxyethylenated (20 EO) sorbitan monopalmitate polyglyceryl-4 isostearate

Advantageously, the anionic surfactant combined may be chosen from:

alkyl sulfates especially C6-C24, even C8-C20, such as dodecyl sulfate (not used as the only surfactant but always combined with a non-ionic surfactant) or decyl sulfate

acylsarcosinates whose acyl group includes 10 to 22 carbon atoms and more particularly a linear acyl group including from 10 to 16 carbon atoms, particularly the sodium salt of N-lauroyl sarcosine;

alkyl ether sulfates, especially C6-C24, even C12-C22, preferably comprising from 2 to 25 ethylene oxide units, more preferably 2 to 10 ethylene oxide units, such as lauryl ether sulfate comprising from 2 to 25 ethylene oxide units.

As combinations of anionic surfactant and non-ionic surfactant, mention may be made of:

polyoxyethylenated (20 EO) sorbitan monopalmitate/sodium decyl sulfate (especially according to 90/10 or 50/50 weight ratio)

polyoxyethylenated (20 EO) sorbitan monopalmitate/sodium N-lauroyl sarcosinate (especially 90/10)

Laurylethersulfate (2 EO)/polyoxyethylenated (20 EO) sorbitan monopalmitate (especially 10/90 or 50/50)

Laurylethersulfate (2 EO)/polyglyceryl-4 isostearate (especially 10/90)

Laurylethersulfate (2 EO)/polyglyceryl-4 isostearate (especially 50/50)

According to a second embodiment of the invention, the surfactant system is one or more cationic surfactant(s).

The cationic surfactant is advantageously chosen from optionally polyoxyalkylenated primary, secondary or tertiary fatty amine salts, quaternary ammonium salts, and mixtures thereof.

As quaternary ammonium salts, mention may be made especially of:

- quaternary ammonium salts having formula (Ia):

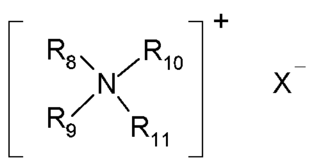

(Ia)

in which:

groups $R_8$ to $R_{11}$, which may be identical or different, represent a linear or branched aliphatic group containing from

1 to 30 carbon atoms, or an aromatic group such as aryl or alkylaryl, at least one of the groups $R_8$ to $R_{11}$ including from 8 to 30 carbon atoms and preferably from 12 to 24 carbon atoms, it being possible for the linear or branched aliphatic groups to include heteroatoms such as, especially, oxygen, nitrogen, sulfur, these heteroatoms not being adjacent, and halogens; and

- $X^-$ is an anion chosen especially from the group of halides such as bromides, chlorides, iodides, fluorides, phosphates, acetates, lactates, $(C_1$-$C_4)$alkyl sulfates, $(C_1$-$C_4)$alkyl sulfonates or $(C_1$-$C_4)$alkylaryl sulfonates;
  $C_1$-$C_{30}$ alkyl, $C_1$-$C_{30}$ alkoxy, $(C_2$-$C_6)$polyoxyalkylene, $C_1$-$C_{30}$ alkylamide, $(C_{12}$-$C_{22})$alkyl-$(C_2C_6)$alkylamido, $(C_{12}$-$C_{22})$alkyl acetate and $C_1$-$C_{30}$ hydroxyalkyl groups;

Mention may be made especially of tetraalkylammonium halides, especially chlorides, such as dialkyldimethylammonium or alkyltrimethylammonium chlorides in which the alkyl group comprises from 12 to 22 carbon atoms, in particular from 14 to 20 carbon atoms such as behenyltrimethylammonium chloride, distearyldimethylammonium chloride, cetyltrimethylammonium chloride (or cetrimonium chloride) and benzyldimethylstearylammonium chloride.

Mention may also be made of palmitylamidopropyltrimethylammonium or stearamidopropyldimethyl-(myristyl acetate)-ammonium halides, and especially chlorides, especially the product sold under the name Ceraphyl® 70 by the company Van Dyk.

Preferably, cationic surfactants having formula (Ia) are chosen from alkyltrimethylammonium halides whose alkyl group includes from 12 to 22 carbon atoms, more preferably from 14 to 20 carbon atoms and more particularly alkyltrimethylammonium chlorides such as behenyltrimethylammonium chloride and cetyltrimethylammonium chloride.

- quaternary ammonium salts of imidazoline having formula (IIa):

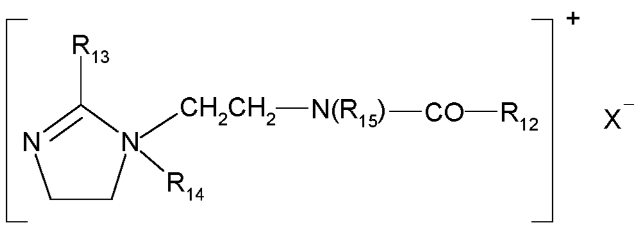

(IIa)

wherein

$R_{12}$ represents an alkenyl or alkyl group comprising from 8 to 30 carbon atoms, for example derived from tallow fatty acids,
$R_{13}$ represents a hydrogen atom, a $C_1$-$C_4$ alkyl group or an alkenyl or alkyl group comprising from 8 to 30 carbon atoms,
$R_{14}$ represents a $C_1$-$C_4$ alkyl group,
$R_{15}$ represents a hydrogen atom or a $C_1$-$C_4$ alkyl group,
$X^-$ is an anion chosen especially from the group of halides, phosphates, acetates, lactates, $(C_1$-$C_4)$alkyl sulfates, and $(C_1$-$C_4)$alkyl- or $(C_1$-$C_4)$alkylarylsulfonates;

Preferably, $R_{12}$ and $R_{13}$ denote a mixture of alkenyl or alkyl groups comprising from 12 to 21 carbon atoms, for example derived from tallow fatty acids, $R_{14}$ denotes a methyl group and $R_{15}$ denotes a hydrogen atom. Such a product is sold, for example, under the name Rewoquat® W75 or W90 by the company Evonik.

- di- or triquaternary ammonium salts having formula (IIIa):

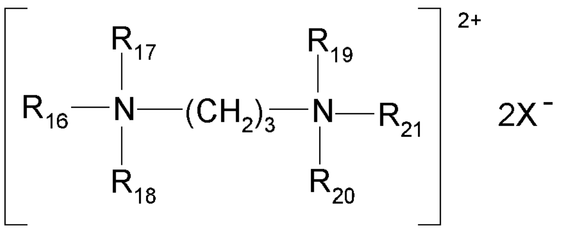

(IIIa)

in which:

- $R_{16}$ denotes an alkyl group comprising from 16 to 30 carbon atoms, which is optionally hydroxylated and/or optionally interrupted with one or more oxygen atoms,
- $R_{17}$ denotes hydrogen, an alkyl group comprising from 1 to 4 carbon atoms or a group $-(CH_2)_3-N^+(R_{16a})(R_{17a})(R_{18a})$; $R_{16a}$, $R_{17a}$ and $R_{18a}$, which may be identical or different, denoting hydrogen or an alkyl group comprising from 1 to 4 carbon atoms,
- $R_{18}$, $R_{19}$, $R_{20}$ and $R_{21}$, which may be identical or different, denote hydrogen or an alkyl group comprising from 1 to 4 carbon atoms, and
- $X^-$ is an anion, chosen especially from the group of halides, acetates, phosphates, nitrates, $(C_1-C_4)$alkyl sulfates and $(C_1-C_4)$alkyl- and $(C_1-C_4)$alkylarylsulfonates, in particular methyl sulfate and ethyl sulfate;

Such compounds are, for example, Finquat CT-P (Quaternium 89) and Finquat CT (Quaternium 75), sold by the company Finetex.

- quaternary ammonium salts containing one or more ester functions, having formula (IVa) below:

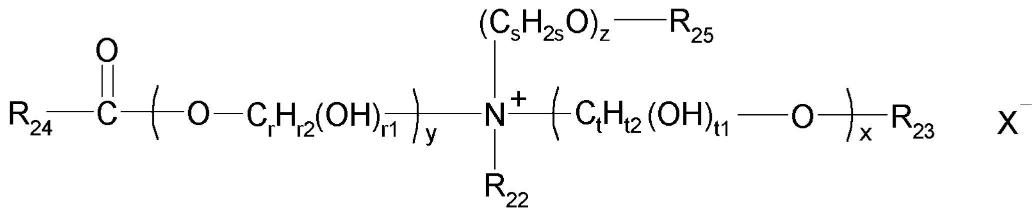

(IVa)

in which:

- $R_{22}$ is chosen from $C_1-C_6$ alkyl groups and $C_1-C_6$ hydroxyalkyl or dihydroxyalkyl groups,
- $R_{23}$ is chosen from the group $R_{26}-C(=O)-$; linear or branched, saturated or unsaturated $C_1-C_{22}$ hydrocarbon-based groups $R_{27}$; and a hydrogen atom,
- $R_{25}$ is chosen from the group $R_{28}-C(=O)-$; linear or branched, saturated or unsaturated $C_1-C_6$ hydrocarbon-based groups $R_{29}$; and a hydrogen atom,
- $R_{24}$, $R_{26}$ and $R_{28}$, which may be identical or different, are chosen from saturated or unsaturated, linear or branched $C_7-C_{21}$ hydrocarbon-based groups,
- r, s and t, which may be identical or different, are integers ranging from 2 to 6,
- r1 and t1, which may be identical or different, are equal to 0 or 1,
- y is an integer ranging from 1 to 10,
- x and z, which may be identical or different, are integers ranging from 0 to 10,
- $X^-$ is an anion,
  it being understood that r2 + r1 = 2r and t1 + t2 = 2t, and that the sum x + y + z ranges from 1 to 15,
  with the proviso that when x = 0 then $R_{23}$ denotes $R_{27}$ and that when z = 0 then $R_{25}$ denotes $R_{29}$.

The alkyl groups $R_{22}$ may be linear or branched, preferably linear. Preferably, $R_{22}$ denotes a methyl, ethyl, hydroxyethyl or dihydroxypropyl group, and more particularly a methyl or ethyl group.
Advantageously, the sum x + y + z ranges from 1 to 10.
When $R_{23}$ is a hydrocarbon-based group $R_{27}$, it may comprise from 12 to 22 carbon atoms, or else may comprise from 1 to 3 carbon atoms.
When $R_{25}$ is a hydrocarbon-based group $R_{29}$, it preferably contains 1 to 3 carbon atoms.
Advantageously, $R_{24}$, $R_{26}$ and $R_{28}$, which may be identical or different, are chosen from linear or branched, saturated or unsaturated $C_{11}-C_{21}$ hydrocarbon-based groups, and more particularly from linear or branched $C_{11}-C_{21}$ alkyl and alkenyl groups.
Preferably, x and z, which may be identical or different, are equal to 0 or 1. Advantageously, y is equal to 1.
Preferably, r, s and t, which may be identical or different, are equal to 2 or 3, and even more particularly are equal to 2.
The anion $X^-$ is preferably a halide, preferably chloride, bromide or iodide, a $(C_1-C_4)$alkyl sulfate, a $(C_1-C_4)$alkylsulfonate or a $(C_1-C_4)$alkylarylsulfonate, a methanesulfonate, a phosphate, a nitrate, a tosylate, an anion derived from an organic acid such as an acetate or a lactate or any other anion that is compatible with the ammonium bearing an ester function. The anion $X^-$ is more particularly a chloride, a methyl sulfate or an ethyl sulfate.
Use is made more particularly of the ammonium salts having formula (VII) in which:

- $R_{22}$ denotes a methyl or ethyl group,
- x and y are equal to 1,
- z is equal to 0 or 1,
- r, s and t are equal to 2,
- $R_{23}$ is chosen from the group $R_{26}$-C(=O)-, methyl, ethyl or $C_{14}$-$C_{22}$ hydrocarbon-based groups; and a hydrogen atom,
- $R_{25}$ is chosen from the group $R_{28}$-C(=O)-; and a hydrogen atom,
- $R_{24}$, $R_{26}$ and $R_{28}$, which may be identical or different, are chosen from linear or branched, saturated or unsaturated $C_{13}$-$C_{17}$ hydrocarbon-based groups, and preferably from linear or branched, saturated or unsaturated $C_{13}$-$C_{17}$ alkyl and alkenyl groups.

Advantageously, the hydrocarbon-based groups are linear.

Among the compounds having formula (IVa), mention may be made of salts, especially the chloride or methyl sulfate of diacyloxyethyldimethylammonium, diacyloxyethylhydroxyethylmethylammonium, monoacyloxyethyldihydroxyethylmethylammonium, triacyloxyethylmethylammonium or monoacyloxyethylhydroxyethyldimethylammonium, and mixtures thereof. The acyl groups preferably contain 14 to 18 carbon atoms and are obtained more particularly from a plant oil such as palm oil or sunflower oil. When the compound contains several acyl groups, these groups may be identical or different.

These products are obtained, for example, by direct esterification of triethanolamine, triisopropanolamine, alkyldiethanolamine or alkyldiisopropanolamine, which are optionally oxyalkylenated, with fatty acids or with fatty acid mixtures especially of plant or animal origin, or by transesterification of the methyl esters thereof. This esterification may be followed by a quaternization by means of an alkylating agent such as an alkyl halide, preferably methyl or ethyl halide, a dialkyl sulfate, preferably dimethyl or diethyl sulfate, methyl methanesulfonate, methyl para-toluenesulfonate, glycol chlorohydrin or glycerol chlorohydrin. Such compounds are sold, for example, under the names Dehyquart® by the company Henkel, Stepanquat® by the company Stepan, Noxamium® by the company CECA or Rewoquat® WE 18 by the company Evonik.

The mixture of cationic surfactants may contain, for example, a mixture of quaternary ammonium monoester, diester and triester salts with a weight majority of diester salts. Use may also be made of the ammonium salts containing at least one ester functional group that are described in patents US-A-4 874 554 and US-A-4 137 180. Use may also be made of behenoylhydroxypropyltrimethylammonium chloride, for example, sold by the company Kao under the name Quartamin BTC 131.

Preferably, the ammonium salts containing at least one ester function contain two ester functions.

More preferably, cationic surfactants used according to the invention are chosen from those having formula (Ia), among alkyltrimethylammonium salts whose alkyl group includes from 12 to 22 carbon atoms, more preferably from 14 to 20 carbon atoms and more particularly behenyltrimethylammonium salts, cetrimonium slats and particularly cetyltrimethylammonium chloride, behenyltrimethylammonium chloride or their mixtures.

The surfactant system may also comprise a non-ionic surfactant as described hereinafter in combination with the cationic surfactant as described previously. The non-ionic surfactant is preferably chosen from:

polyoxyethylenated lauryl alcohol 4 EO (Laureth-4), polyoxyethylenated lauryl alcohol 23 EO (Laureth-23),
polyoxyethylenated (20 EO) sorbitan monopalmitate
polyglyceryl-4 isostearate.

As combinations of cationic surfactant and non-ionic surfactant, mention may be made of:

cetrimonium chloride/polyoxyethylenated sorbitan monopalmitate (20 EO) (especially 10/90)
behenyltrimethylammonium chloride/polyoxyethylenated sorbitan monopalmitate (20 EO) (especially 10/90)
cetrimonium chloride/polyglyceryl-4 isostearate (especially 10/90)
behenyl trimethylammonium chloride/polyglyceryl-4 isostearate (especially 10/90)

According to a third embodiment of the invention, the surfactant system is one or more non-ionic surfactants.

The non-ionic surfactant may be chosen from alcohols and alpha-diols, these compounds being polyethoxylated and/or polypropoxylated and/or polyglycerolated, the number of ethylene oxide and/or propylene oxide groups possibly ranging from 2 to 100, and the number of glycerol groups possibly ranging from 2 to 30; these compounds comprising at least one fatty chain comprising from 8 to 30 carbon atoms and especially from 16 to 30 carbon atoms;

Mention may also be made of polyethoxylated fatty amides preferably having from 2 to 30 ethylene oxide units, polyglycerolated fatty amides including on average from 1 to 5, and in particular from 1.5 to 4, glycerol groups; polyoxyethylenated fatty acid esters of sorbitan having preferably from 2 to 40 units of ethylene oxide, fatty acid esters of sucrose, polyoxyalkylenated and preferably polyoxyethylenated fatty acid esters containing from 2 to 150 mol of ethylene

oxide, such as oxyethylenated plant oils.

Mention may also be made of non-ionic surfactants of alkyl(poly)glycoside type, represented especially by the following general formula: $R_1O-(R_2O)_t-(G)_v$ in which:

- $R_1$ represents a linear or branched alkyl or alkenyl substituent comprising 6 to 24 carbon atoms and especially 8 to 18 carbon atoms, or an alkylphenyl substituent whose linear or branched alkyl substituent comprises 6 to 24 carbon atoms and especially 8 to 18 carbon atoms;
- $R_2$ represents an alkylene substituent comprising 2 to 4 carbon atoms,
- G represents a sugar unit comprising 5 to 6 carbon atoms,
- t denotes a value ranging from 0 to 10 and preferably 0 to 4,
- v denotes a value ranging from 1 to 15 and preferably 1 to 4.

Preferably, the alkyl(poly)glycoside surfactants are compounds of the formula described above in which:

- $R_1$ denotes a linear or branched, saturated or unsaturated alkyl substituent comprising from 8 to 18 carbon atoms,
- $R_2$ represents an alkylene substituent comprising 2 to 4 carbon atoms,
- t denotes a value ranging from 0 to 3 and preferably equal to 0,
- G denotes glucose, fructose or galactose, preferably glucose;
- the degree of polymerization, i.e. the value of v, possibly ranging from 1 to 15 and preferably from 1 to 4; the mean degree of polymerization more particularly being between 1 and 2.

The glucoside bonds between the sugar units are generally of 1-6 or 1-4 type and preferably of 1-4 type. Preferably, the alkyl(poly)glycoside surfactant is an alkyl(poly)glucoside surfactant. $C_8/C_{16}$ alkyl(poly)glucosides 1,4, and in particular decyl glucosides and caprylyl/capryl glucosides, are most particularly preferred.

Among commercial products, mention may be made of the products sold by the company Cognis under the names Plantaren® (600 CS/U, 1200 and 2000) or Plantacare® (818, 1200 and 2000); the products sold by the company SEPPIC under the names Oramix CG 110 and Oramix® NS 10; the products sold by the company BASF under the name Lutensol GD 70, or else the products sold by the company Chem Y under the name AG10 LK.

Preferably, use is made of $C_8/C_{16}$-alkyl (poly)glycosides 1,4, in particular as an aqueous 53% solution, such as those sold by Cognis under the reference Plantacare® 818 UP.

Preferably, the non-ionic surfactants are chosen from polyoxyethylenated C8-C30 fatty acid esters (preferably C12-C18) of sorbitan, polyethoxylated C8-C30 (preferably C12-18) fatty alcohols, polyglycerolated C8-C30 (preferably C12-C18) fatty acid esters, polyoxyethylenated compounds having preferably from 2 to 30 moles of ethylene oxide, polyglycerolated compounds having preferably from 2 to 16 moles of glycerol;
and mixtures thereof.

The polyoxyethylenated C8-C30 fatty alcohols may be chosen from C12-C18 fatty alcohols, in particular polyoxyethylenated lauryl alcohol, cetyl alcohol, myristyl alcohol, and stearyl alcohol having from 2 to 30 mol of ethylene oxide, such as:

cetyl alcohol polyoxyethylenated with 2 EO (Ceteth-2) (HLB 5.3)
cetyl alcohol polyoxyethylenated with 6 EO (Ceteth-6) (HLB 11.1)
cetyl alcohol polyoxyethylenated with 10 EO (Ceteth-10) (HLB 12.9)
cetyl alcohol polyoxyethylenated with 20 EO (Ceteth-20) (HLB 15.7)
cetyl alcohol polyoxyethylenated with 24 EO (Ceteth-24) (HLB 16.3)
lauryl alcohol polyoxyethylenated with 2 EO (Laureth-2) (HLB 6.1)
lauryl alcohol polyoxyethylenated with 3 EO (Laureth-3) (HLB 8)
lauryl alcohol polyoxyethylenated with 4 EO (Laureth-4) (HLB 9.4)
lauryl alcohol polyoxyethylenated with 7 EO (Laureth-7) (HLB 12.3)
lauryl alcohol polyoxyethylenated with 9 EO (Laureth-9) (HLB 13.6)
lauryl alcohol polyoxyethylenated with 10 EO (Laureth-10) (HLB 13.9)
lauryl alcohol polyoxyethylenated with 12 EO (Laureth-12) (HLB 14.6)
lauryl alcohol polyoxyethylenated with 21 EO (Laureth-21) (HLB 15.5)
lauryl alcohol polyoxyethylenated with 23 EO (Laureth-23) (HLB 16.3)
stearyl alcohol polyoxyethylenated with 2 EO (Steareth-2) (HLB 4.9)
stearyl alcohol polyoxyethylenated with 10 EO (Steareth-10) (HLB 12.4)
stearyl alcohol polyoxyethylenated with 20 EO (Steareth-20) (HLB 15.2)
stearyl alcohol polyoxyethylenated with 21 EO (Steareth-21) (HLB 15.5)

The polyoxyethylenated C8-C30 fatty acid esters (preferably C12-C18) of sorbitan may be chosen from polyoxyethylenated esters of C12-C18 fatty acids, in particular lauric, myristic, cetylic or stearic acids, of sorbitan especially containing from 2 to 30 mol of ethylene oxide, such as:

polyoxyethylenated sorbitan monolaurate (4 EO) (Polysorbate-21) (HLB 13.3)
polyoxyethylenated sorbitan monolaurate (20 EO) (Polysorbate-20) (HLB 16.7)
polyoxyethylenated sorbitan monopalmitate (20 EO) (Polysorbate-40) (HLB 15.6)
polyoxyethylenated sorbitan monostearate (20 EO) (Polysorbate-60) (HLB 14.9)
polyoxyethylenated sorbitan monostearate (4 EO) (Polysorbate-61) (HLB 9.6)
polyoxyethylenated sorbitan monooleate (20 EO) (Polysorbate-80) (HLB 15)

The polyglycerolated C8-C30 fatty acid esters may be chosen from polyglycerolated esters of C12-C18 fatty acids, in particular lauric, myristic, palmitic, stearic or isostearic acid, having from 2 to 16 mol of glycerol, such as:

polyglyceryl-2 laurate, polyglyceryl-3 laurate, polyglyceryl-4 laurate, polyglyceryl-5 laurate, polyglyceryl-6 laurate, polyglyceryl-10 laurate;
polyglyceryl-2 myristate, polyglyceryl-3 myristate, polyglyceryl-4 myristate, polyglyceryl-5 myristate, polyglyceryl-6 myristate, polyglyceryl-10 myristate;
polyglyceryl-2 palmitate, polyglyceryl-3 palmitate, polyglyceryl-6 palmitate, polyglyceryl-10 palmitate;
polyglyceryl-2 isostearate, polyglyceryl-3 isostearate, polyglyceryl-4 isostearate,
polyglyceryl-5 isostearate, polyglyceryl-6 isostearate, polyglyceryl-10 isostearate;
polyglyceryl-2 stearate, polyglyceryl-3 stearate, polyglyceryl-4 stearate, polyglyceryl-5 stearate, polyglyceryl-6 stearate, polyglyceryl-8 stearate, polyglyceryl-10 stearate.

The non-ionic surfactant is preferably chosen from polyoxyethylenated lauryl alcohol (4 EO), polyoxyethylenated lauryl alcohol (23 EO), oxyethylene sorbitan monopalmitate (20 EO), polyglyceryl-4 isostearate.

When the surfactant system comprises a non-ionic surfactant and an anionic or cationic surfactant, said non-ionic surfactant may have any HLB at all.

When the surfactant system comprises only a non-ionic surfactant, the surfactant then has an HLB greater than 10, and preferably greater than or equal to 15.

When the surfactant system comprises only a mixture of non-ionic surfactants, said mixture then has an HLB greater than 10, and preferably greater than or equal to 15.

The term HLB is well known to those skilled in the art, and denotes the hydrophilic-lipophilic balance of a surfactant at 25°C in the Griffin sense.

The term "hydrophilic-lipophilic balance (HLB)" is intended to mean the equilibrium between the size and the strength of the hydrophilic group and the size and the strength of the lipophilic group of the surfactant. This HLB value according to Griffin is defined in J. Soc. Cosm. Chem. 1954 (volume 5), pages 249-256.

Surfactants having an HLB greater than 10 can be used that are cited in
the reference work McCutcheons Emulsifiers & Detergents, International Edition, 1998 and following.

Reference may also be made to Kirk-Othmer's Encyclopedia of Chemical Technology, volume 22, pp. 333-432, 3rd edition, 1979, Wiley, for the definition of the emulsifying properties and functions of surfactants, in particular pp. 347-377 of this reference, for non-ionic surfactants.

The HLB of a surfactant mixture containing a% of A and b% of B (mass percentage) is calculated as follows:

$$HLB\ (A + B) = a\%\ (HLB\ A) + b\ \%\ (HLB\ B)$$

As an example of non-ionic surfactants with HLB greater than 10, the surfactants described previously can be used, and preferably chosen from oxyethylene sorbitan monopalmitate (20 EO) (HLB = 15.3), Laureth-23 (HLB = 16.3) and Laureth-9 (13.6),

As examples of non-ionic surfactants with HLB less than or equal to 10, mention may be made for example of polyglyceryl-4 isostearate (HLB = 5), Laureth-4 (HLB = 9.4), Laureth-2 (HLB = 6.1) and Laureth-3 (HLB = 8) .

As non-ionic surfactant mixture, mention may be made of the mixture of polyoxyethylenated lauryl alcohol 4 EO and polyoxyethylenated lauryl alcohol 23 EO.

The polyalkene supramolecular polymer may be present in the aqueous dispersion in a content ranging from 2% to 50% by weight and preferably ranging from 5% to 40% by weight, relative to the total weight of the dispersion.

The surfactant system may be present in the aqueous composition in a content ranging from 0.01% to 5% by weight, especially ranging from 0.02% to 4% by weight, preferably ranging from 0.03% to 3% by weight, more preferably

ranging from 0.04% to 2% by weight, relative to the total weight of the dispersion.

Advantageously, the polyalkene supramolecular polymer and the surfactant system are present in the aqueous dispersion according to a polymer/surfactant weight ratio ranging from 9 to 49, preferably ranging from 9 to 40, preferably ranging from 9 to 35.

A subject of the invention is also a process for preparing the aqueous dispersion described previously, comprising the following steps:

(i) a synthesis step of the polyalkene supramolecular polymer in an organic solvent S like for example the 2-methyl tetrahydrofuran, ethyl acetate;

(ii) then an addition step:

either of an aqueous solution containing the surfactant system,
or of an organic solution containing the organic solvent S and the surfactant system then water addition;

(iii) then a step of dispersion of the mixture obtained under stirring, especially at a rate ranging from 3000 to 30000 rpm, especially for a duration ranging from 1 to 60 minutes preferably ranging from 5 to 15 minutes;

(iv) then a step of evaporation of the organic solvent S.

The step of dispersion may be conducted with stirring using a disperser, for example with a rotor/stator such as an Ultra Turax, for example at the rate of 24,000 rpm.

The invention also relates to a composition comprising the aqueous dispersion of the polyalkene supramolecular polymer described previously.

The composition advantageously comprises a physiologically acceptable medium, i.e. a medium compatible with the keratin fibres and/or materials of a living creature, in particular of humans, such as, for example, in a non-limiting manner, the skin, the lips, the nails, the hair, the eyelashes or the eyebrows.

The supramolecular polymer may be present in the composition according to the invention in a content ranging from 0.1% to 50% by weight, preferably ranging from 0.5% to 40% by weight, more preferably ranging from 0.5% to 30% by weight, relative to the total weight of the composition.

The composition according to the invention may comprise at least one cosmetic ingredient chosen from colorants, fillers, oils, waxes, pastes, (additional) surfactants, UV filters, cosmetic actives; fragrances, propellants, film-forming polymers (additional, especially different from the polymer of the aqueous dispersion), thickeners, preservatives.

The composition according to the invention may be a composition for making up especially the skin and/or the lips and/or the lashes. The composition may be a product for the complexion such as a foundation, a blusher or an eyeshadow; a lip product such as a lipstick or a lipcare product or a gloss; a concealer product; a blush; an eyeliner; a lipstick pencil or eye pencil; a body makeup product; a mascara.

The composition may also be a skin or lip care product; a hygiene product, a sun protection product.

The composition may also be a haircare composition, especially a shampoo, a conditioner, a styling product, a hair treatment product, a shaping product.

The invention is illustrated in greater detail in the following examples.

**Polymer 1:** described in example 2 of FR-A-2938760

106.1 g of dihydroxylated hydrogenated 1,2-polybutadiene polymer (GI2000 from Nisso, Mn=2100) is heated in the presence of 22 mg of catalyst (dibutyltin dilaurate) at 80°C, under reduced pressure, for two hours. The temperature of the mixture is reduced to 20°C, under argon, followed by addition of 10 ml of isododecane and 19.3 g of isophorone diisocyanate (IPDI). The mixture is stirred for 16 hours at 20°C under a controlled atmosphere, and is then heated to 120°C, followed by addition of 25 ml of propylene carbonate. 12 g of 6-methylisocytosine is added, resulting in a homogeneous white suspension. This suspension is heated to 140°C and stirred at this temperature for 6 hours. The reaction is monitored by infrared spectroscopy, up to the total disappearance of the characteristic peak for isocyanates (2250 $cm^{-1}$). The mixture is then reduced to 30°C, and 400 ml of heptane, 200 ml of THF and 50 ml of ethanol are added, followed by filtration through Celite. The mixture is then stripped with isododecane.

A solution of the polymer in isododecane, with a solids content of 20%, is finally obtained; the polymer is characterized by GPC (Mn = 7000 and polydispersity index = 2.05).

**Polymer P2:** synthesis

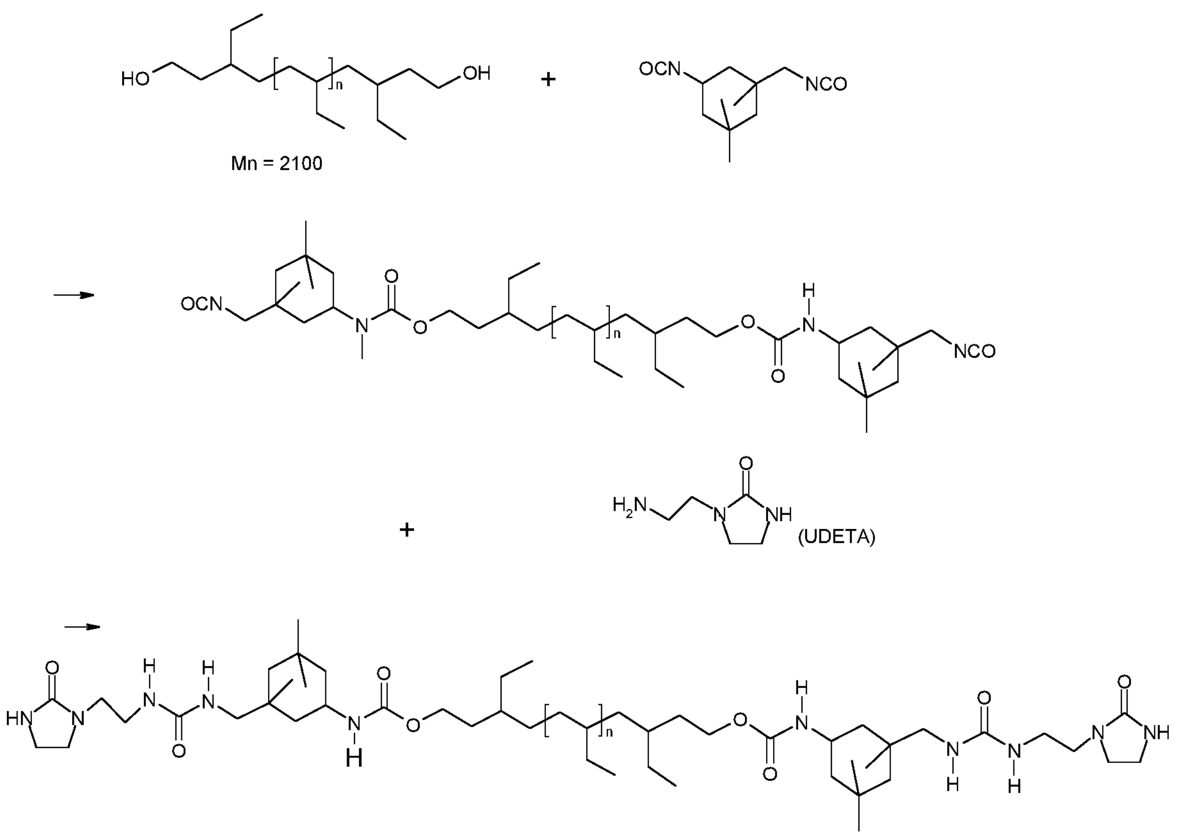


To a reactor under an argon atmosphere, 200 g of dihydroxylated hydrogenated 1,2-polybutadiene polymer (GI 2000 from Nisso; Mn=2100), 50 g of isododecane and 36.1 g of isophorone diisocyanate were added. The solution was heated to 40 °C, then 63 μl of catalyst (dibutyltin dilaurate) was added, holding the temperature between 38°C and 42°C for three hours. Next 19.25 g of 2-aminoethylimidazolidin-2-one (UDETA) was added and the medium was heated to 120°C for three hours. Then the temperature in the reactor was lowered to 70°C and 100 ml of ethanol was added. The mixture was left to react for 1 hour. 350 g of isododecane was added and the ethanol was removed by distillation. A solution containing 38.5% by weight of polymer in isododecane was thus obtained.

The polymer obtained has a number-average molecular weight (Mn) of 4600 and a weight-average molecular weight (Mw) of 10400. (analysis by GPC)

**Examples of aqueous polymer dispersions:**

**Example of Dispersion 1:**

To a jacketed reactor set at the temperature of 15 °C, 50 g of solution of polymer 1 at 10 % by weight in 2-methyl tetrahydrofuran (2-Me THF) was added. Then with stirring using an Ultra-Turax at 24000 rpm for 10 min, an aqueous solution made by mixing 0.4 g of sodium lauryl ether sulfate (2 EO) and 50 g of water was added. The white solution obtained was then evaporated under vacuum to remove the 2 Me-THF.

Accordingly, we obtained an aqueous dispersion of Polymer 1 which is homogeneous, white and stable after one week's storage and one month's storage at ambient temperature (23 °C). This dispersion has solids content of 18%.

Other aqueous dispersions of Polymer 1 and Polymer 2 with other surfactants described in Table 1 hereinafter according to the procedure described previously were prepared (surfactant content for sodium N-lauroyl sarcosinate of 2.8 g). To use the surfactant, it can be put in solution in 50 g of 2-Me THF in the place of water as specified in the table, then 50 g of water is added.

For each aqueous dispersion prepared, the stability was evaluated after one month's storage at ambient temperature (23 °C)

Comparative Examples 1 to 11: Aqueous dispersions with anionic surfactant system

| Example | Polymer | Surfactant system (put in solution in) | Polymer/ surfactant ratio | Solids content of the dispersion |
|---|---|---|---|---|
| 1 | Polymer 1 | Sodium N-lauroyl sarcosinate (water) | 97/3 | 20.4% |
| 2 | Polymer 1 | Sodium lauryl ether sulfate 2 EO (water) | 91/9 | 18% |
| 3 | Polymer 1 | Sodium lauryl ether sulfate 2 EO (water)/ polyoxyethylenated sorbitan monopalmitate (20 EO) (2-MeTHF) 50/50 | 91/9 | 25% |
| 4 | Polymer 1 | Sodium lauryl ether sulfate 2 EO (water)/ polyoxyethylenated sorbitan monopalmitate (20 EO) (2-Me-THF) 10/90 | 91/9 | 20% |
| 5 | Polymer 1 | Sodium lauryl ether sulfate 2 EO (water)/ polyglyceryl-4 isostearate (2 Me-THF) 50/50 | 91/9 | 15% |
| 6 | Polymer 1 | Mixture of polyoxyethylenated sorbitan monopalmitate (20 EO)/sodium dodecyl sulfate (90/10) | 91/9 | 11% |
| 7 | Polymer 1 | Mixture of polyoxyethylenated sorbitan monopalmitate (20 EO)/sodium dodecyl sulfate (50/50) | 91/9 | 13% |
| 8 | Polymer 1 | Sodium lauryl ether sulfate 2 EO (water) polyglyceryl-4 isostearate (2 Me-THF) 10/90 | 91/9 | 10.8% |
| 9 | Polymer 1 | Sodium decyl sulfate (water) | 91/9 | 15% |
| 10 | Polymer 2 | Sodium N-lauroyl sarcosinate (water) | 97/3 | 24% |
| | | | | |
| 11 (outside the invention) | Polymer 1 | Sodium dodecyl sulfate (water) | 91/9 | unstable |

Comparative Examples 12 to 17: Aqueous dispersions with cationic surfactant (invention) or amphoteric surfactant (outside the invention)

| Example | Polymer | Surfactant system (put in solution in) | Polymer/s urfactant ratio | Solids content of the dispersion |
|---|---|---|---|---|
| 12 | Polymer 1 | Behenyltrimethylammonium chloride (water) | 91/9 | 21% |
| 13 | Polymer 1 | Cetrimonium chloride (water) | 97/3 | 26.5% |
| 14 | Polymer 1 | behenyl trimethyl ammonium chloride (water)/ polyoxyethylenated sorbitan monopalmitate (20 EO) (water) (10/90) | 91/9 | 14% |
| 15 | Polymer 1 | cetrimonium chloride (water)/polyoxyethylenated sorbitan monopalmitate (20 EO) (water) (10/90) | 91/9 | 16% |

(continued)

| Example | Polymer | Surfactant system (put in solution in) | Polymer/s urfactant ratio | Solids content of the dispersion |
|---|---|---|---|---|
| 16 | Polymer 1 | cetrimonium chloride (water)/ polyglyceryl-4 isostearate (water) (10/90) | 91/9 | 26% |
| | | | | |
| 17 (outside the invention) | Polymer 1 | cocobetain (water) | 91/9 | unstable |

**Comparison examples 18 to 22:** aqueous dispersions with non-ionic surfactant

| Example | Polymer | Surfactant system (put in solution in) | Polymer/s urfactant ratio | Solids content of the dispersion |
|---|---|---|---|---|
| 18 | Polymer 1 | polyoxyethylenated sorbitan monopalmitate (2 Me THF)<br>HLB = 15.3 | 91/9 | 18% |
| 19 | Polymer 1 | polyoxyethylenated lauryl alcohol (23 EO) (water)/<br>polyoxyethylenated lauryl alcohol (4 EO) (95/5) (2 MeTHF)<br>HLB = 16 | 91/9 | 34% |
| 20 | Polymer 1 | polyoxyethylenated lauryl alcohol (23 EO) (water)/<br>polyoxyethylenated lauryl alcohol (4 EO) (2MeTHF) (80/20)<br>HLB = 15.1 | 91/9 | 18.5% |
| | | | | |
| 21 (outside the invention) | Polymer 1 | polyoxyethylenated lauryl alcohol (4 EO) HLB = 10 (2 MeTHF) | 91/9 | unstable |
| 22 (outside the invention) | Polymer 1 | polyglyceryl-4 isostearate (2 Me-THF) HLB = 5 | 91/9 | unstable |

**Evaluation of the low-tack appearance of the polymer films**

The low-tack appearance of the polymer films obtained from aqueous dispersions of Examples 1 (Polymer 1) and 10 (Polymer 2) was evaluated by adjusting by dilution with water to a polymer content of 20% by weight.

For comparison, a solution of Polymer 1 and a solution of Polymer 2 at 10% by weight in isododecane were also prepared.

The aqueous dispersions were spread on glass plates then left to dry for 7 days at ambient temperature (23 °C) whereas the organic solutions were spread on contrast card then left to dry for 24 hours at ambient temperature (23 °C). The films prepared were 30 $\mu$m thick after drying.

Next, for each film, tack (expressed in N) was measured using the automated platform (Freeslate/Symyx Core module) to determine the detachment force exerted by the film during the removal phase of a flexible Viton ball 10 mm in diameter in contact with the film in the following conditions: rate of approach and removal of the ball: 5 mm/s, ball-film contact time: 5 s, contact force exerted by the ball: 1 N.

The following results were obtained:

| | Tack (N) |
|---|---|
| Polymer dispersion 1 (ex 1) | 0.02 |
| Polymer solution 1 in isododecane | 0.12 |
| Polymer dispersion 2 (ex 10) | 0.12 |
| Polymer 2 solution in isododecane | 0.55 |

The results show that the aqueous dispersions of Examples 1 and 10 according to the invention form a film having lower tack than that of the film obtained from solutions of the same polymer in isododecane.

So the aqueous dispersion of polymer produces a less tacky polymer film.

**Example 23:**

The following composition is prepared (% by weight):

| | |
|---|---|
| Aqueous dispersion of Example 1 | 10% |
| Xanthan gum | 2% |
| Fragrance qs | |
| Water qs | 100% |

The composition applied to the skin forms a non-tacky film.

**Example 24:**

The following skin makeup composition is prepared (% by weight):

| | |
|---|---|
| Aqueous dispersion of Example 10 | 10% |
| Xanthan gum | 2% |
| Fragrance qs | |
| Water-soluble dye | qs |
| Water qs | 100% |

The composition applied to the skin forms a coloured non-tacky film.

**Example 25:**

The following hair composition is prepared (% by weight):

| | |
|---|---|
| Aqueous dispersion of Example 1 | 10% |
| Xanthan gum | 2% |
| Fragrance qs | |
| Water qs | 100% |

The composition applied to the hair forms a coating, non-tacky film.

**Example 26:**

The following mascara composition is prepared (% by weight):

| | |
|---|---|
| Aqueous dispersion of Example 1 | 10% |
| Xanthan gum | 3% |
| Black iron oxides | qs |
| Water qs | 100% |

The composition applied to the lashes forms a coating, non-tacky film.

## Claims

1. Aqueous dispersion of supramolecular polyalkene polymer particles with a surfactant system chosen from:

   1) at least one anionic surfactant optionally combined with at least one non-ionic surfactant, with the exclusion of the surfactant system containing only dodecyl sulfate and/or an alkali metal salt of dodecyl sulfate;
   2) at least one cationic surfactant, optionally combined with at least one non-ionic surfactant;
   3) at least one non-ionic surfactant having an HLB greater than 10 or a mixture of non-ionic surfactants, said mixture having an HLB greater than 10, without being combined with an ionic surfactant;
   being understood that the polyalkene supramolecular polymer is a polymer including in its structure at least one polyalkene portion and at least one portion including at least one group that can form at least three hydrogen bonds, preferably four hydrogen bonds.

2. Dispersion according to the preceding claim, **characterized in that** the supramolecular polyalkene polymer can be obtained from the condensation of at least one polyalkene polymer functionalized with at least one reactive group, with at least one junction group functionalized with at least one reactive group that can react with the reactive group(s) of the functionalized polyalkene polymer, said junction group being capable of forming at least three H (hydrogen) bonds, preferably four hydrogen bonds.

3. Dispersion according to Claim 2, **characterized in that** the functionalized polyalkene polymer has the formula:

   HX-P-X'H

   - where XH and X'H are reactive groups, with X and X', which may be identical or different, chosen from O, S, NH and $NR_a$, $R_a$ representing a linear or branched $C_1$-$C_6$ alkyl group;
   - P represents a homo- or copolymer that can be obtained by polymerization of one or more linear, cyclic and/or branched, mono- or polyunsaturated $C_2$-$C_5$ alkenes.

4. Dispersion according to Claim 3, **characterized in that** P represents a polyethylene, a polybutylene, a polybutadiene, a hydrogenated polybutadiene, a polyisoprene, a poly(1,3-pentadiene), a polyisobutylene, and copolymers thereof, and preferably a hydrogenated polybutadiene.

5. Dispersion according to one of Claims 3 or 4, **characterized in that** X = X' = O.

6. Dispersion according to one of Claims 2 to 5, **characterized in that** the functionalized junction group has the formula:

   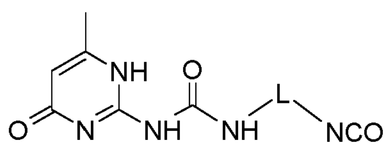

   or

   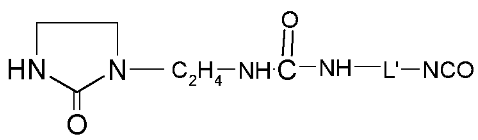

   in which L is a saturated or unsaturated, or even aromatic, linear, cyclic and/or branched C1-C20 divalent (alkylene) carbon-based group, optionally comprising 1 to 4 N and/or O heteroatoms.

7. Dispersion according to the previous claim, **characterized in that** L is a divalent substituent chosen from phenylene, 1,2-ethylene, 1,6-hexylene, 1,4-butylene, 1,6-(2,4,4-trimethylhexylene), 1,4-(4-methylpentylene), 1,5-(5-methylhexylene), 1,6-(6-methylheptylene), 1,5-(2,2,5-trimethylhexylene), 1,7-(3,7-dimethyloctylene), -isophorone-, 4,4'-methylene bis(cyclohexylene), tolylene, 2-methyl-1,3-phenylene, 4-methyl-1,3-phenylene, 4,4-biphenylenemethylene; preferably a divalent group: phenylene, -isophorone-, 4,4'-methylene biscyclohexylene, tolylene, 2-methyl-1,3-phenylene, 4-methyl-1,3-phenylene, 4,4-biphenylenemethylene;
   preferably -isophorone-, 4,4'-methylene biscyclohexylene, 2-methyl-1,3-phenylene; more preferably -isophorone-.

8. Dispersion according to any one of the preceding claims, **characterized in that** the supramolecular polymer corresponds to the formula:

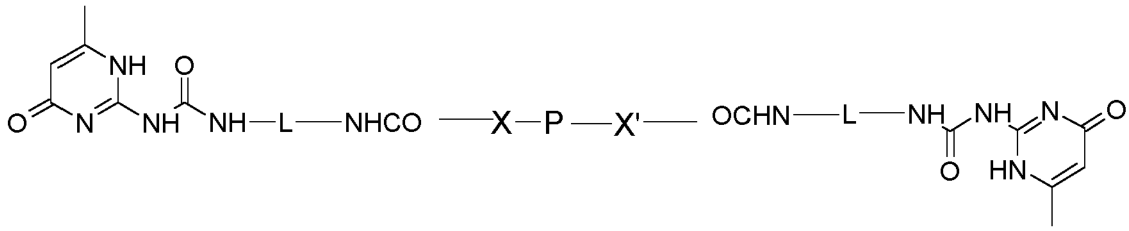

(C1)

or formula (C2):

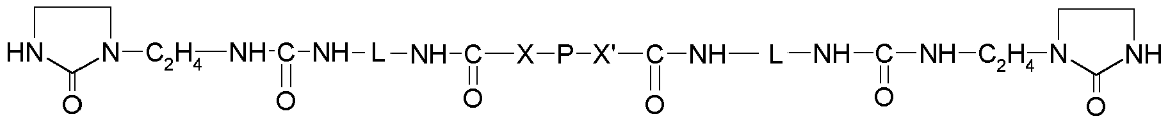

(C2)

P, X, X', L having the meanings as defined in Claims 3 to 6.

**9.** Dispersion according to one of the preceding claims, **characterized in that** the surfactant system comprises at least one anionic surfactant, with the exclusion of the surfactant system comprising only dodecyl sulfate and/or an alkali metal salt of dodecyl sulfate.

**10.** Dispersion according to one of the preceding claims, **characterized in that** the anionic surfactant is chosen from sulfate surfactants, sulfonate surfactants, carboxylic surfactants, and mixtures thereof.

**11.** Dispersion according to one of the preceding claims, **characterized in that** the anionic surfactant is chosen from acylglycinates, acyllactylates, acylsarcosinates, acylglutamates; alkyl-D-galactosideuronic acids, alkyl ether carboxylic acids, alkyl(C6-30 aryl) ether carboxylic acids, alkylamido ether carboxylic acids; and the salts of these compounds;
the alkyl and/or acyl groups of these compounds including from 6 to 30 carbon atoms, especially from 10 to 22, better still from 10 to 16 carbon atoms; where the aryl group preferably denotes a phenyl or benzyl group;
where these compounds may be polyoxyethylenated;
preferably chosen from acylsarcosinates and acylglycinates whose acyl group includes 10 to 22 carbon atoms and more particularly a linear acyl group including from 10 to 16 carbon atoms, particularly the sodium salt of N-lauroyl sarcosine and sodium N-cocoyl glycinate;
preferably chosen from acylsarcosinates whose acyl group includes 10 to 22 carbon atoms and more particularly a linear acyl group including from 10 to 16 carbon atoms, particularly the sodium salt of N-lauroyl sarcosine.

**12.** Aqueous dispersion according to one of the preceding claims, **characterized in that** the anionic surfactant is chosen from
alkyl sulfates, alkyl ether sulfates, alkylamido ether sulfates, alkylaryl polyether sulfates, monoglyceride sulfates; and also the salts of these compounds;
the alkyl groups of these compounds including from 6 to 30 carbon atoms, in particular from 12 to 28, better still from 14 to 24 or even from 16 to 22 carbon atoms; where the aryl group preferably denotes a phenyl or benzyl group;
where these compounds may be polyoxyethylenated;
preferably chosen from:

- alkyl sulfates especially C6-C24, even C8-C20, such as dodecyl sulfate (but not used as the only surfactant but always combined with a non-ionic surfactant) or decyl sulfate
- alkyl ether sulfates, especially C6-C24, even C12-C22, preferably comprising from 2 to 25 ethylene oxide

units, more preferably 2 to 10 ethylene oxide units, such as lauryl ether sulfate comprising from 2 to 25 ethylene oxide units particularly in the form of alkali metal or alkaline earth metal, ammonium, or aminoalcohol salts, more particularly in the form of alkali metal salts such as sodium salts; preferably chosen from sodium laureth sulfate and sodium decyl sulfate.

**13.** Dispersion according to one of Claims 1 to 8, **characterized in that** the surfactant system comprises a cationic surfactant.

**14.** Dispersion according to the preceding claim, **characterized in that** the cationic surfactant is chosen from quaternary ammonium salts having formula (Ia):

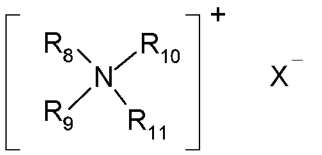

(Ia)

in which:

groups $R_8$ to $R_{11}$, which may be identical or different, represent a linear or branched aliphatic group containing from 1 to 30 carbon atoms, or an aromatic group such as aryl or alkylaryl, at least one of the groups $R_8$ to $R_{11}$ including from 8 to 30 carbon atoms and preferably from 12 to 24 carbon atoms, it being possible for the linear or branched aliphatic groups to include heteroatoms such as, especially, oxygen, nitrogen, sulfur, these heteroatoms not being adjacent, and halogens; and

- $X^-$ is an anion chosen especially from the group of halides such as bromides, chlorides, iodides, fluorides, phosphates, acetates, lactates, $(C_1$-$C_4)$alkyl sulfates, $(C_1$-$C_4)$alkyl sulfonates or $(C_1$-$C_4)$alkylaryl sulfonates; $C_1$-$C_{30}$ alkyl, $C_1$-$C_{30}$ alkoxy, $(C_2$-$C_6)$polyoxyalkylene, $C_1$-$C_{30}$ alkylamide, $(C_{12}$-$C_{22})$alkyl-$(C_2C_6)$alkylamido, $(C_{12}$-$C_{22})$alkyl acetate and $C_1$-$C_{30}$ hydroxyalkyl groups;

preferably chosen from tetraalkylammonium halides, especially chlorides, such as dialkyldimethylammonium or alkyltrimethylammonium chlorides in which the alkyl group comprises from 12 to 22 carbon atoms, in particular from 14 to 20 carbon atoms such as behenyltrimethylammonium chloride, distearyldimethylammonium chloride, cetyltrimethylammonium chloride (or cetrimonium chloride) and benzyldimethylstearylammonium chloride; palmitylamidopropyltrimethylammonium or stearamidopropyldimethyl-(myristyl acetate)-ammonium halides, especially chlorides;

preferably chosen from alkyltrimethylammonium halides whose alkyl group includes from 12 to 22 carbon atoms, more preferably from 14 to 20 carbon atoms and more particularly alkyltrimethylammonium chlorides such as behenyltrimethylammonium chloride and cetyltrimethylammonium chloride.

**15.** Dispersion according to one of the preceding claims, **characterized in that** the surfactant system comprises a non-ionic surfactant.

**16.** Dispersion according to any one of the preceding claims, **characterized by** the fact that the non-ionic surfactant is chosen from alcohols, alpha-diols, these compounds being polyethoxylated and/or polypropoxylated and/or polyglycerolated, the number of ethylene oxide and/or propylene oxide groups possibly ranging from 2 to 100, and the number of glycerol groups possibly ranging from 2 to 30; these compounds comprising at least one fatty chain including from 8 to 30 carbon atoms, especially from 16 to 30 carbon atoms; polyethoxylated fatty amides preferably having from 2 to 30 ethylene oxide units, polyglycerolated fatty amides including on average from 1 to 5, and in particular from 1.5 to 4 glycerol groups; ethoxylated fatty acid esters of sorbitan having preferably from 2 to 40 units of ethylene oxide, fatty acid esters of sucrose, polyoxyalkylenated and preferably polyoxyethylenated fatty acid esters containing from 2 to 150 mol of ethylene oxide;

preferably chosen from polyoxyethylenated C8-C30 fatty acid esters (preferably C12-C18) of sorbitan, polyoxyethylenated C8-C30 (preferably C12-18) fatty alcohols, polyglycerolated C8-C30 (preferably C12-C18) fatty acid esters, polyoxyethylenated compounds having preferably from 2 to 30 moles of ethylene oxide, polyglycerolated compounds having preferably from 2 to 16 moles of glycerol;

and mixtures thereof.

**17.** Dispersion according to one of Claims 1 to 8, **characterized in that** the surfactant system comprises a non-ionic surfactant having HLB greater than 10, preferably chosen from

cetyl alcohol polyoxyethylenated with 6 EO
cetyl alcohol polyoxyethylenated with 10 EO
cetyl alcohol polyoxyethylenated with 20 EO
cetyl alcohol polyoxyethylenated with 24 EO
lauryl alcohol polyoxyethylenated with 7 EO
lauryl alcohol polyoxyethylenated with 9 EO
lauryl alcohol polyoxyethylenated with 10 EO
lauryl alcohol polyoxyethylenated with 12 EO
lauryl alcohol polyoxyethylenated with 23 EO
stearyl alcohol polyoxyethylenated with 10 EO
stearyl alcohol polyoxyethylenated with 20 EO
stearyl alcohol polyoxyethylenated with 21 EO
polyoxyethylenated sorbitan monolaurate (4 EO)
polyoxyethylenated sorbitan monolaurate (20 EO)
polyoxyethylenated sorbitan monopalmitate (20 EO)
polyoxyethylenated sorbitan monostearate (20 EO)
polyoxyethylenated sorbitan monooleate (20 EO)

**18.** Dispersion according to one of Claims 1 to 8, **characterized in that** the surfactant system comprises a surfactant with HLB less than or equal to 10, preferably chosen from:

cetyl alcohol polyoxyethylenated with 2 EO
lauryl alcohol polyoxyethylenated with 2 EO
lauryl alcohol polyoxyethylenated with 3 EO
lauryl alcohol polyoxyethylenated with 4 EO
stearyl alcohol polyoxyethylenated with 2 EO
sorbitan monostearate polyoxyethylenated with 4 EO
polyglyceryl-4 isostearate
and preferably chosen from
polyglyceryl-4 isostearate, lauryl alcohol polyoxyethylenated with 2 EO, lauryl alcohol polyoxyethylenated with 3 EO, lauryl alcohol polyoxyethylenated with 4 EO.

**19.** Dispersion according to one of Claims 1 to 8, **characterized in that** the surfactant system comprises a mixture of non-ionic surfactant of polyoxyethylenated lauryl alcohol (4 EO) and polyoxyethylenated lauryl alcohol (23 EO).

**20.** Dispersion according to one of Claims 1 to 8, **characterized in that** the surfactant system comprises a combination of anionic surfactant and non-ionic surfactant chosen from:

polyoxyethylenated sorbitan monopalmitate (20 EO)/sodium decyl sulfate
polyoxyethylenated sorbitan monopalmitate (20 EO)/sodium N-lauroyl sarcosinate
Laurylethersulfate/polyoxyethylenated sorbitan monopalmitate (20 EO)
Laurylethersulfate/polyglyceryl-4 isostearate
Laurylethersulfate/polyglyceryl-4 isostearate

**21.** Dispersion according to one of Claims 1 to 8, **characterized in that** the surfactant system comprises a combination of cationic surfactant and non-ionic surfactant chosen from:

cetrimonium chloride/polyoxyethylenated sorbitan monopalmitate (20 EO)
behenyl trimethylammonium chloride/polyoxyethylenated sorbitan monopalmitate (20 EO)
cetrimonium chloride/polyglyceryl-4 isostearate
behenyl trimethylammonium chloride/polyglyceryl-4 isostearate.

**22.** Dispersion according to one of the preceding claims, **characterized in that** the supramolecular polymer is present in a content ranging from 2% to 50% by weight and preferably ranging from 5% to 40% by weight, relative to the total weight of the dispersion.

**23.** Dispersion according to one of the preceding claims, **characterized in that** the surfactant system is present in a content ranging from 0.01% to 5% by weight, relative to the total weight of the dispersion, especially from 0.02% to

4% by weight, preferably ranging from 0.03% to 3% by weight, more preferably from 0.04% to 2% by weight.

**24.** Dispersion according to one of the preceding claims, **characterized in that** the supramolecular polymer and the surfactant system are present in an polymer/surfactant weight ratio ranging from 9 to 49, preferably ranging from 9 to 40, more preferably ranging from 9 to 35.

**25.** Process for preparing an aqueous dispersion of polymer as defined according to one of Claims 1 to 24, comprising the following steps:

(i) a synthesis step of the polyalkene supramolecular polymer in an organic solvent S like 2-methyl tetrahydrofuran, ethyl acetate;
(ii) then an addition step:
either of an aqueous solution containing the surfactant system,
or of an organic solution containing the organic solvent S and the surfactant system then water addition;
(iii) then a step of dispersion of the mixture obtained under stirring, especially for a duration ranging from 1 to 60 minutes preferably ranging from 5 to 15 minutes;
(iv) then a step of evaporation of the organic solvent S,

the surfactant system being as defined according to one of Claims 1 and 8 to 20.

**26.** Composition comprising an aqueous dispersion of polyalkene supramolecular polymer according to one of Claims 1 to 24.

**27.** Composition according to the preceding claim, **characterized in that** it comprises a physiologically acceptable medium.

**28.** Composition according to any one of Claims 26 or 27, in which the polymer is present in a content ranging from 0.1% to 50% by weight, preferably ranging from 0.5% to 40% by weight and more preferably ranging from 0.5% to 30% by weight relative to the total weight of the composition.

**29.** Composition according to one of Claims 26 to 28, **characterized in that** it comprises at least one cosmetic ingredient chosen from colorants, fillers, oils, waxes, pastes, (additional) surfactants, UV filters, cosmetic actives; fragrances, propellants, film-forming polymers (additional, especially different from the polymer of the aqueous dispersion), thickeners, preservatives.

**30.** Cosmetic process for treating keratin materials comprising the application to keratin materials of a composition according to any one of Claims 26 to 29.

**31.** Process according to the preceding claim, **characterized in that** it is a cosmetic process for caring for or making up keratin materials.

## Patentansprüche

**1.** Wässrige Dispersion von Teilchen von supramolekularem Polyalkenpolymer mit einem Tensidsystem, das aus

1) mindestens einem anionischen Tensid, gegebenenfalls kombiniert mit mindestens einem nichtionischen Tensid, unter Ausschluss des Tensidsystems, das nur Dodecylsulfat und/oder ein Alkalimetallsalz von Dodecylsulfat enthält;
2) mindestens einem kationischen Tensid, gegebenenfalls kombiniert mit mindestens einem nicht ionischen Tensid;
3) mindestens einem nichtionischen Tensid mit einem HLB-Wert von mehr als 10 oder einer Mischung von nichtionischen Tensiden, wobei die Mischung einen HLB-Wert von mehr als 10 aufweist, ohne Kombination mit einem ionischen Tensid ausgewählt ist;

wobei es sich versteht, dass es sich bei dem supramolekularen Polyalkenpolymer um ein Polymer handelt, das in seiner Struktur mindestens einen Polyalkenteil und mindestens einen Teil mit mindestens einer Gruppe, die mindestens drei Wasserstoffbrückenbindungen, vorzugsweise vier Wasserstoffbrückenbindungen, bilden kann, enthält.

2. Dispersion nach dem vorhergehenden Anspruch, **dadurch gekennzeichnet, dass** das supramolekulare Polyalkenpolymer aus der Kondensation von mindestens einem mit mindestens einer reaktiven Gruppe funktionalisierten Polyalkenpolymer mit mindestens einer Verknüpfungsgruppe, die mit mindestens einer reaktiven Gruppe, die mit der reaktiven Gruppe bzw. den reaktiven Gruppen des funktionalisierten Polyalkenpolymers reagieren können, funktionalisiert ist, erhältlich ist, wobei die Verknüpfungsgruppe zur Bildung von mindestens drei H-Brückenbindungen (Wasserstoffbrückenbindungen), vorzugsweise vier Wasserstoffbrückenbindungen, befähigt ist.

3. Dispersion nach Anspruch 2, **dadurch gekennzeichnet, dass** das funktionalisierte Polyalkenpolymer die Formel

HX-P-X'H

aufweist,

- wobei XH und X'H, die gleich oder verschieden sein können, aus O, S, NH und $NR_a$ ausgewählt sind, wobei $R_a$ für eine lineare oder verzweigte $C_1$-$C_6$-Alkylgruppe steht;
- P für ein Homo- oder Copolymer steht, das durch Polymerisation von einem oder mehreren linearen, cyclischen und/oder verzweigten, einfach oder mehrfach ungesättigten $C_2$-$C_5$-Alkenen erhältlich ist.

4. Dispersion nach Anspruch 3, **dadurch gekennzeichnet, dass** P für ein Polyethylen, ein Polybutylen, ein Polybutadien, ein hydriertes Polybutadien, ein Polyisopren, ein Poly(1,3-pentadien), ein Polyisobutylen und Copolymere davon und vorzugsweise ein hydriertes Polybutadien steht.

5. Dispersion nach Anspruch 3 oder 4, **dadurch gekennzeichnet, dass** X = X' = O.

6. Dispersion nach einem der Ansprüche 2 bis 5, **dadurch gekennzeichnet, dass** die funktionalisierte Verknüpfungsgruppe die Formel

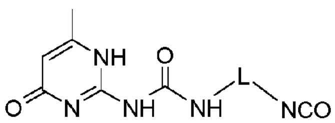

oder

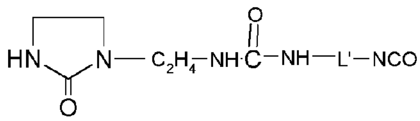

aufweist, wobei L für eine gesättigte oder ungesättigte oder sogar aromatische, lineare, cyclische und/oder verzweigte zweiwertige C1-C20-Gruppe auf Kohlenwasserstoffbasis (Alkylengruppe), die gegebenenfalls 1 bis 4 N- und/oder O-Heteroatome umfasst, steht.

7. Dispersion nach dem vorhergehenden Anspruch, **dadurch gekennzeichnet, dass** L für einen zweiwertigen Substituenten, der aus Phenylen, 1,2-Ethylen, 1,6-Hexylen, 1,4-Butylen, 1,6-(2,4,4-Trimethylhexylen), 1,4-(4-Methylpentylen), 1,5-(5-Methylhexylen), 1,6-(6-Methylheptylen), 1,5-(2,2,5-Trimethylhexylen), 1,7-(3,7-Dimethyloctylen), -Isophoron-, 4,4'-Methylenbis(cyclohexylen), Tolylen, 2-Methyl-1,3-phenylen, 4-Methyl-1,3-phenylen, 4,4-Biphenylenmethylen ausgewählt ist;
vorzugsweise -Isophoron-, 4,4'-Methylenbiscyclohexylen, 2-Methyl-1,3-phenylen;
weiter bevorzugt -Isophoron-;
steht.

8. Dispersion nach einem der vorhergehenden Ansprüche, **dadurch gekennzeichnet, dass** das supramolekulare Polymer der Formel

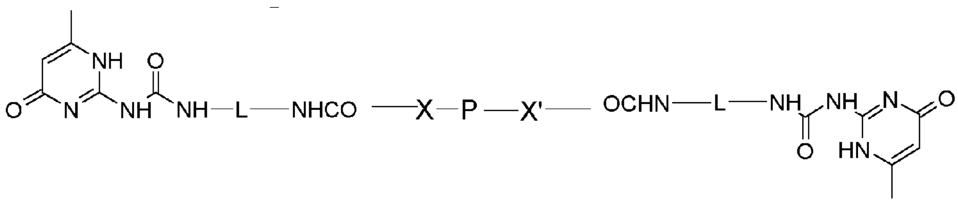

(C1)

oder der Formel (C2):

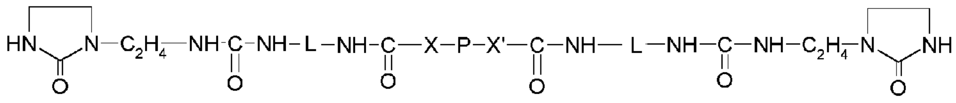

(C2)

entspricht, wobei P, X, X' und L die wie in den Ansprüchen 3 bis 6 definierten Bedeutungen haben.

**9.** Dispersion nach einem der vorhergehenden Ansprüche, **dadurch gekennzeichnet, dass** das Tensidsystem mindestens ein anionisches Tensid umfasst, unter Ausschluss des Tensidsystems, das nur Dodecylsulfat und/oder ein Alkalimetallsalz von Dodecylsulfat enthält.

**10.** Dispersion nach einem der vorhergehenden Ansprüche, **dadurch gekennzeichnet, dass** das anionische Tensid aus Sulfat-Tensiden, Sulfonat-Tensiden, Carboxyl-Tensiden und Mischungen davon ausgewählt ist.

**11.** Dispersion nach einem der vorhergehenden Ansprüche, **dadurch gekennzeichnet, dass** das anionische Tensid ausgewählt ist aus
Acylglycinaten, Acyllactylaten, Acylsarcosinaten, Acylglutamaten; Alkyl-D-galactosiduronsäuren, Alkylethercarbonsäuren, Alkyl(C6-C30-aryl)ethercarbonsäuren, Alkylamidoethercarbonsäuren und den Salzen dieser Verbindungen;
wobei die Alkyl- und/oder Acylgruppen dieser Verbindungen 6 bis 30 Kohlenstoffatome, speziell 10 bis 22 und noch besser 10 bis 16 Kohlenstoffatome enthalten; wobei die Arylgruppe vorzugsweise eine Phenyl- oder Benzylgruppe bedeutet;
wobei diese Verbindungen polyoxyethyleniert sein können;
vorzugsweise ausgewählt aus Acylsarcosinaten und Acylglycinaten, deren Acylgruppe 10 bis 22 Kohlenstoffatome und spezieller eine lineare Acylgruppe mit 10 bis 16 Kohlenstoffatomen umfasst, insbesondere dem Natriumsalz von N-Lauroylsarcosin und Natrium-N-cocoylglycinat;
vorzugsweise ausgewählt aus Acylsarcosinaten, deren Acylgruppe 10 bis 22 Kohlenstoffatome und spezieller eine lineare Acylgruppe mit 10 bis 16 Kohlenstoffatomen umfasst, insbesondere dem Natriumsalz von N-Lauroylsarcosin.

**12.** Wässrige Dispersion nach einem der vorhergehenden Ansprüche, **dadurch gekennzeichnet, dass** das anionische Tensid ausgewählt ist aus
Alkylsulfaten, Alkylethersulfaten, Alkylamidoethersulfaten, Alkylarylpolyethersulfaten, Monoglyceridsulfaten sowie den Salzen dieser Verbindungen;
wobei Alkylgruppen dieser Verbindungen 6 bis 30 Kohlenstoffatome, insbesondere 12 bis 28, noch besser 14 bis 24 oder sogar 16 bis 22 Kohlenstoffatome enthalten; wobei die Arylgruppe vorzugsweise eine Phenyl- oder Benzylgruppe bedeutet;
wobei diese Verbindungen polyoxyethyleniert sein können;
vorzugsweise ausgewählt aus

- Alkylsulfaten, insbesondere C6-C24 oder sogar C8-C20, wie Dodecylsulfat (aber nicht als einziges Tensid verwendet, sondern immer mit einem nicht ionischen Tensid kombiniert) oder Decylsulfat,
- Alkylethersulfaten, insbesondere C6-C24 oder sogar C12-C22, vorzugsweise mit 2 bis 25 Ethylenoxideinheiten, weiter bevorzugt 2 bis 10 Ethylenoxideinheiten, wie Laurylethersulfat mit 2 bis 25 Ethylenoxideinheiten;

insbesondere in Form von Alkalimetall- oder Erdalkalimetall-, Ammonium- oder Aminoalkoholsalzen, spezieller in Form von Alkalimetallsalzen wie Natriumsalzen;
vorzugsweise ausgewählt aus Natriumlaurethsulfat und Natriumdecylsulfat.

**13.** Dispersion nach einem der Ansprüche 1 bis 8, **dadurch gekennzeichnet, dass** das Tensidsystem ein kationisches Tensid umfasst.

**14.** Dispersion nach dem vorhergehenden Anspruch, **dadurch gekennzeichnet, dass** das kationische Tensid aus quaternären Ammoniumsalzen der Formel (Ia) :

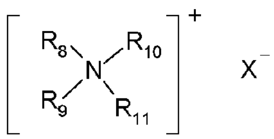

(Ia)

ausgewählt ist, wobei:

die Gruppen $R_8$ bis $R_{11}$, die gleich oder verschieden sein können, für eine lineare oder verzweigte aliphatische Gruppe mit 1 bis 30 Kohlenstoffatomen oder eine aromatische Gruppe wie Aryl oder Alkylaryl stehen, wobei mindestens eine der Gruppen $R_8$ bis $R_{11}$ 8 bis 30 Kohlenstoffatome und vorzugsweise 12 bis 24 Kohlenstoffatome enthält, wobei die linearen oder verzweigten aliphatischen Gruppen Heteroatome wie insbesondere Sauerstoff, Stickstoff, Schwefel, wobei diese Heteroatome nicht benachbart sind, und Halogene enthalten können; und
$X^-$ ein Anion ist, das insbesondere aus der Gruppe der Halogenide wie Bromide, Chloride, Iodide, Fluoride, Phosphate, Acetate, Lactate, ($C_1$-$C_4$)-Alkylsulfate, ($C_1$-$C_4$) -Alkylsulfonate oder ($C_1$-$C_4$)-Alkylarylsulfonate ausgewählt ist;
$C_1$-$C_{30}$-Alkyl-, $C_1$-$C_{30}$-Alkoxy-, ($C_2$-$C_6$) -Polyoxyalkylen-, $C_1$-$C_{30}$-Alkylamid-, ($C_{12}$-$C_{22}$) -Alkyl- ($C_2$-$C_6$)-alkylamido-, ($C_{12}$-$C_{22}$) -Alkylacetat- und $C_1$-$C_{30}$-Hydroxyalkylgruppen;
vorzugsweise ausgewählt aus Tetraalkylammoniumhalogeniden, insbesondere -chloriden, wie Dialkyldimethylammonium- oder Alkyltrimethylammoniumchloriden, in denen die Alkylgruppe 12 bis 22 Kohlenstoffatome und insbesondere 14 bis 20 Kohlenstoffatome umfasst, wie Behenyltrimethylammoniumchlorid, Distearyldimethylammoniumchlorid, Cetyltrimethylammoniumchlorid (oder Cetrimoniumchlorid) und Benzyldimethylstearylammoniumchlorid; Palmitylamidopropyltrimethylammonium- oder Stearamidopropyldimethyl(myristylacetat)ammoniumhalogeniden, insbesondere -chloriden;
vorzugsweise ausgewählt aus Alkyltrimethylammoniumhalogeniden, deren Alkylgruppe 12 bis 22 Kohlenstoffatome und weiter bevorzugt 14 bis 20 Kohlenstoffatome enthält, und spezieller Alkyltrimethylammoniumchloride wie Behenyltrimethylammoniumchlorid und Cetyltrimethylammoniumchlorid.

**15.** Dispersion nach einem der vorhergehenden Ansprüche, **dadurch gekennzeichnet, dass** das Tensidsystem ein nichtionisches Tensid umfasst.

**16.** Dispersion nach einem der vorhergehenden Ansprüche, **dadurch gekennzeichnet, dass** das nichtionische Tensid ausgewählt ist aus Alkoholen, alpha-Diolen, wobei diese Verbindungen polyethoxyliert und/oder polypropoxyliert und/oder polyglyceriniert sind, wobei die Zahl der Ethylenoxid- und/oder Propylenoxidgruppen im Bereich von 2 bis 100 liegen kann und die Zahl der Glyceringruppen im Bereich von 2 bis 30 liegen kann; wobei diese Verbindungen mindestens eine Fettkette mit 8 bis 30 Kohlenstoffatomen und insbesondere 16 bis 30 Kohlenstoffatomen enthalten; polyethoxylierten Fettamiden, vorzugsweise mit 2 bis 30 Ethylenoxideinheiten, polyglycerinierten Fettamiden mit durchschnittlich 1 bis 5 und insbesondere 1,5 bis 4 Glyceringruppen; ethoxylierten Fettsäureestern von Sorbitan mit vorzugsweise 2 bis 40 Ethylenoxideinheiten, Fettsäureestern von Saccharose, polyoxyalkylenierten und vorzugsweise polyoxyethylenierten Fettsäureestern mit 2 bis 150 mol Ethylenoxid;
vorzugsweise ausgewählt aus polyoxyethylenierten C8-C30-Fettsäureestern (vorzugsweise C12-C18-Fettsäureestern) von Sorbitan, polyoxyethylenierten C8-C30-Fettalkoholen (vorzugsweise C12-C18-Fettalkoholen), polyglycerinierten C8-C30-Fettsäureestern (vorzugsweise C12-C18-Fettsäureestern), polyoxyethylenierten Verbindungen mit vorzugsweise 2 bis 30 mol Ethylenoxid, polyglycerinierten Verbindungen mit vorzugsweise 2 bis 16 mol Glycerin;
und Mischungen davon.

**17.** Dispersion nach einem der Ansprüche 1 bis 8, **dadurch gekennzeichnet, dass** das Tensidsystem ein nichtionisches Tensid mit einem HLB-Wert von mehr als 10 umfasst, vorzugsweise ausgewählt aus
mit 6 EO polyoxyethyleniertem Cetylalkohol,
mit 10 EO polyoxyethyleniertem Cetylalkohol,
mit 20 EO polyoxyethyleniertem Cetylalkohol,
mit 24 EO polyoxyethyleniertem Cetylalkohol,
mit 7 EO polyoxyethyleniertem Laurylalkohol,
mit 9 EO polyoxyethyleniertem Laurylalkohol,
mit 10 EO polyoxyethyleniertem Laurylalkohol,
mit 12 EO polyoxyethyleniertem Laurylalkohol,

mit 23 EO polyoxyethyleniertem Laurylalkohol,
mit 10 EO polyoxyethyleniertem Stearylalkohol,
mit 20 EO polyoxyethyleniertem Stearylalkohol,
mit 21 EO polyoxyethyleniertem Stearylalkohol, polyoxyethyleniertem Sorbitanmonolaurat (4 EO), polyoxyethyleniertem Sorbitanmonolaurat (20 EO), polyoxyethyleniertem Sorbitanmonopalmitat (20 EO), polyoxyethyleniertem Sorbitanmonostearat (20 EO), polyoxyethyleniertem Sorbitanmonooleat (20 EO).

**18.** Dispersion nach einem der Ansprüche 1 bis 8, **dadurch gekennzeichnet, dass** das Tensidsystem ein Tensid mit einem HLB-Wert von weniger oder gleich 10 umfasst, vorzugsweise ausgewählt aus
mit 2 EO polyoxyethyleniertem Cetylalkohol,
mit 2 EO polyoxyethyleniertem Laurylalkohol,
mit 3 EO polyoxyethyleniertem Laurylalkohol,
mit 4 EO polyoxyethyleniertem Laurylalkohol,
mit 2 EO polyoxyethyleniertem Stearylalkohol,
mit 4 EO polyoxyethyleniertem Sorbitanmonostearat, Polyglyceryl-4-isostearat,
und vorzugsweise ausgewählt aus Polyglyceryl-4-isostearat, mit 2 EO polyoxyethyleniertem Laurylalkohol, mit 3 EO polyoxyethyleniertem Laurylalkohol, mit 4 EO polyoxyethyleniertem Laurylalkohol.

**19.** Dispersion nach einem der Ansprüche 1 bis 8, **dadurch gekennzeichnet, dass** das Tensidsystem eine Mischung von nichtionischem Tensid von polyoxyethyleniertem Laurylalkohol (4 EO) und polyoxyethyleniertem Laurylalkohol (23 EO) umfasst.

**20.** Dispersion nach einem der Ansprüche 1 bis 8, **dadurch gekennzeichnet, dass** das Tensidsystem eine Kombination von anionischem Tensid und nichtionischem Tensid umfasst, die ausgewählt ist aus:

polyoxyethyleniertem Sorbitanmonopalmitat (20 EO)/Natriumdecylsulfat,
polyoxyethyleniertem Sorbitanmonopalmitat (20 EO)/Natrium-N-lauroylsarcosinat,
Laurylethersulfat/ polyoxyethyleniertem Sorbitanmonopalmitat (20 EO),
Laurylethersulfat/ Polyglyceryl-4-isostearat,
Laurylethersulfat/ Polyglyceryl-4-isostearat.

**21.** Dispersion nach einem der Ansprüche 1 bis 8, **dadurch gekennzeichnet, dass** das Tensidsystem eine Kombination von kationischem Tensid und nichtionischem Tensid umfasst, die ausgewählt ist aus:

Cetrimoniumchlorid/polyoxyethyleniertem Sorbitanmonopalmitat (20 EO),
Behenyltrimethylammoniumchlorid/polyoxyethyleniertem Sorbitanmonopalmitat (20 EO),
Cetrimoniumchlorid/Polyglyceryl-4-isostearat, Behenyltrimethylammoniumchlorid/Polyglyceryl-4-isostearat.

**22.** Dispersion nach einem der vorhergehenden Ansprüche, **dadurch gekennzeichnet, dass** das supramolekulare Polymer in einem Gehalt im Bereich von 2 bis 50 Gew.-% und vorzugsweise im Bereich von 5 bis 40 Gew.-%, bezogen auf das Gesamtgewicht der Dispersion, vorliegt.

**23.** Dispersion nach einem der vorhergehenden Ansprüche, **dadurch gekennzeichnet, dass** das Tensidsystem in einem Gehalt im Bereich von 0,01 bis 5 Gew.-%, bezogen auf das Gesamtgewicht der Dispersion, speziell von 0,02 bis 4 Gew.-%, vorzugsweise im Bereich von 0,03 bis 3 Gew.-%, weiter bevorzugt von 0,04 bis 2 Gew.-%, vorliegt.

**24.** Dispersion nach einem der vorhergehenden Ansprüche, **dadurch gekennzeichnet, dass** das supramolekulare Polymer und das Tensidsystem in einem Polymer/Tensid-Gewichtsverhältnis im Bereich von 9 bis 49, vorzugsweise im Bereich von 9 bis 40, weiter bevorzugt im Bereich von 9 bis 35, vorliegen.

**25.** Verfahren zur Herstellung einer wässrigen Polymerdispersion gemäß einem der Ansprüche 1 bis 24, das die folgenden Schritte umfasst:

(i) einen Schritt der Synthese des supramolekularen Polyalkenpolymers in einem organischen Lösungsmittel S wie 2-Methyltetrahydrofuran, Essigsäureethylester;
(ii) dann einen Schritt der Zugabe
entweder einer wässrigen Lösung, die das Tensidsystem enthält,
oder einer organischen Lösung, die das organische Lösungsmittel S und das Tensidsystem enthält, dann

Wasserzugabe;
(iii) dann einen Schritt des Dispergierens der erhaltenen Mischung unter Rühren, insbesondere für eine Dauer im Bereich von 1 bis 60 Minuten, vorzugsweise im Bereich von 5 bis 15 Minuten;
(iv) dann einen Schritt des Verdampfens des organischen Lösungsmittels S,

wobei das Tensidsystem wie gemäß einem der Ansprüche 1 und 8 bis 20 definiert ist.

**26.** Zusammensetzung, umfassend eine wässrige Dispersion von supramolekularem Polyalkenpolymer nach einem der Ansprüche 1 bis 24.

**27.** Zusammensetzung nach dem vorhergehenden Anspruch, **dadurch gekennzeichnet, dass** sie ein physikalisch unbedenkliches Medium umfasst.

**28.** Zusammensetzung nach Anspruch 26 oder 27, wobei das Polymer in einem Gehalt im Bereich von 0,1 bis 50 Gew.-%, vorzugsweise im Bereich von 0,5 bis 40 Gew.-% und weiter bevorzugt im Bereich von 0,5 bis 30 Gew.-%, bezogen auf das Gesamtgewicht der Zusammensetzung, vorliegt.

**29.** Zusammensetzung nach einem der Ansprüche 26 bis 28, **dadurch gekennzeichnet, dass** sie mindestens einen kosmetischen Bestandteil umfasst, der aus Farbmitteln, Füllstoffen, Ölen, Wachsen, Pasten, (zusätzlichen) Tensiden, UV-Filtern, kosmetischen Wirkstoffen; Duftstoffen, Treibmitteln, filmbildenden Polymeren (zusätzlich, insbesondere von dem Polymer der wässrigen Dispersion verschiedene), Verdickern und Konservierungsstoffen ausgewählt ist.

**30.** Kosmetisches Verfahren zur Behandlung von Keratinmaterialien, bei dem man auf die Keratinmaterialien eine Zusammensetzung nach einem der Ansprüche 26 bis 29 aufbringt.

**31.** Verfahren nach dem vorhergehenden Anspruch, **dadurch gekennzeichnet, dass** es sich um ein kosmetisches Verfahren zum Pflegen oder Schminken von Keratinmaterialien handelt.

## Revendications

**1.** Dispersion aqueuse de particules de polymère polyalcène supramoléculaire avec un système tensioactif choisi parmi :

1) au moins un tensioactif anionique éventuellement associé avec au moins un tensioactif non ionique, à l'exclusion du système tensioactif contenant uniquement du dodécyl sulfate et/ou un sel de métal alcalin du dodécyl sulfate ;
2) au moins un tensioactif cationique, éventuellement associé avec au moins un tensioactif non ionique ;
3) au moins un tensioactif non ionique ayant un HLB supérieur à 10 ou un mélange de tensioactifs non ioniques, ledit mélange ayant un HLB supérieur à 10, sans être associé à un tensioactif ionique ;

étant entendu que le polymère polyalcène supramoléculaire est un polymère comprenant dans sa structure au moins une partie polyalcène et au moins une partie comprenant au moins un groupe qui peut former au moins trois liaisons hydrogène, préférablement quatre liaisons hydrogène.

**2.** Dispersion selon la revendication précédente, **caractérisée en ce que** le polymère polyalcène supramoléculaire est susceptible d'être issu de la condensation d'au moins un polymère polyalcène fonctionnalisé par au moins un groupe réactif, avec au moins un groupe de jonction fonctionnalisé par au moins un groupe réactif susceptible de réagir avec le ou les groupes réactifs du polymère polyalcène fonctionnalisé, ledit groupe de jonction étant capable de former au moins 3 liaisons H (hydrogène), de préférence 4 liaisons H.

**3.** Dispersion selon la revendication 2, **caractérisée en ce que** le polymère polyalcène fonctionnalisé est de formule :

HX-P-X'H

- XH et X'H étant des groupes réactifs, avec X et X', identiques ou différents, choisis parmi O, S, NH ou $NR_a$, $R_a$ représentant un groupe alkyle linéaire ou ramifié en $C_1$-$C_6$;

- P représente un homo- ou un copolymère susceptible d'être obtenu par polymérisation d'un ou plusieurs alcènes, linéaires, cycliques et/ou ramifiés, mono ou polyinsaturés, en $C_2$-$C_5$.

**4.** Dispersion selon la revendication 3, **caractérisée en ce que** P représente un polyéthylène, un polybutylène, un polybutadiène, un polybutadiène hydrogéné, un polyisoprène, un poly(1,3-pentadiène), un polyisobutylène, et leurs copolymères, et de préférence un polybutadiène hydrogéné.

**5.** Dispersion selon l'une des revendications 3 et 4, **caractérisée en ce que** X = X' = O.

**6.** Dispersion selon l'une des revendications 2 à 5, **caractérisée en ce que** le groupe de jonction fonctionnalisé est de formule :

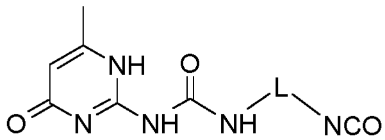

ou

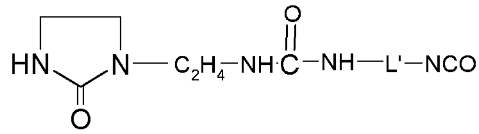

dans laquelle L est un groupe carboné divalent (alkylène), saturé ou insaturé, voire aromatique, en C1-C20, linéaire, cyclique et/ou ramifié, comprenant éventuellement 1 à 4 hétéroatomes N et/ou O.

**7.** Dispersion selon la revendication précédente, **caractérisée en ce que** L est un radical divalent choisi parmi : phénylène ; 1,2-éthylène ; 1,6-héxylène ; 1,4-butylène ; 1,6-(2,4,4-triméthylhexylène) ; 1,4-(4-méthylpentylène) ; 1,5-(5-méthylhexylène) ; 1,6-(6-méthylheptylène) ; 1,5-(2,2,5-triméthylhexylène) ; 1,7-(3,7-diméthyloctylène) ; -isophorone- ; 4,4'-méthylène bis(cyclohexylène) ; tolylène ; 2-méthyl-1,3-phénylène ; 4-méthyl-1,3-phénylène ; 4, 4-biphénylèneméthylène ;
de préférence un groupement divalent phénylène ; -isophorone- ; 4,4'-méthylène biscyclohexylène ; tolylène ; 2-méthyl-1,3-phénylène ; 4-méthyl-1,3-phénylène ; 4,4-biphénylèneméthylène, ;
préférentiellement -isophorone- ; 4,4'-méthylène biscyclohexylène ; 2-méthyl-1,3-phénylène ;
plus préférentiellement -isophorone-.

**8.** Dispersion selon l'une quelconque des revendications précédentes, **caractérisée en ce que** le polymère supramoléculaire répond à la formule :

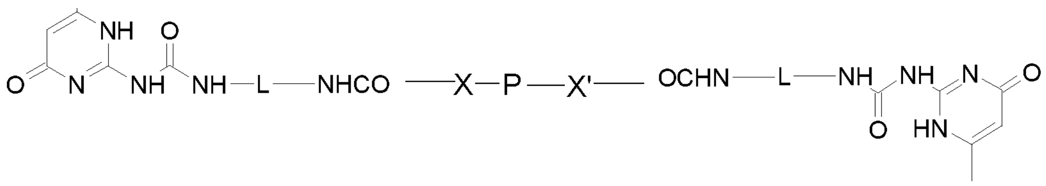

(C1)

ou de formule (C2) :

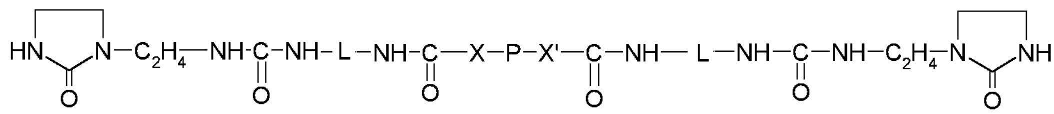

(C2)

P, X, X', L ayant les significations telles que définies dans les revendications 3 à 6.

**9.** Dispersion selon l'une des revendications précédentes, **caractérisée en ce que**
le système tensioactif comprend un ou plusieurs tensioactif(s) anionique(s), à l'exclusion du système tensioactif comprenant uniquement du dodécyl sulfate et/ou un sel de métal alcalin de dodécyl sulfate.

**10.** Dispersion selon l'une des revendications précédentes, **caractérisée en ce que** le tensioactif anionique est choisi parmi les tensioactifs sulfates, les tensioactifs sulfonates, les tensioactifs carboxyliques, et leurs mélanges.

**11.** Dispersion selon l'une des revendications précédentes, **caractérisée** en ce le tensioactif anionique est choisi parmi les acylglycinates, les acyllactylates, les acylsarcosinates, les acylglutamates ; les acides alkyl-D-galactoside-uroniques, les acides alkyléthercarboxyliques, les acides alkyl(aryl en C6-30)éthercarboxyliques, les acides alkylamidoéthercarboxyliques ; ainsi que les sels de ces composés ;
les groupes alkyle et/ou acyle de ces composés comportant de 6 à 30 atomes de carbone, notamment de 10 à 22, encore mieux de 10 à 16 atomes de carbone ; le groupe aryle désignant de préférence un groupe phényle ou benzyle ; ces composés pouvant être polyoxyéthylénés.
de préférence choisi parmi les acylsarcosinates et les acylglycinates dont le groupe acyle renferme de 10 à 22 atomes de carbone et plus particulièrement un groupe acyle linéaire renfermant de 10 à 16 atomes de carbone, en particulier le sel de sodium de la N-lauroyl sarcosine et le N-cocoyle glycinate de sodium ; préférentiellement choisi parmi les acylsarcosinates dont le groupe acyle renferme de 10 à 22 atomes de carbone et plus particulièrement un groupe acyle linéaire renfermant de 10 à 16 atomes de carbone, en particulier le sel de sodium de la N-lauroyl sarcosine.

**12.** Dispersion aqueuse selon l'une des revendications précédentes, **caractérisée en ce que** le tensioactif anionique est choisi parmi
les alkylsulfates, les alkyléthersulfates, les alkylamidoéthersulfates, les alkylarylpolyéthersulfates, les monoglycéride-sulfates; ainsi que les sels de ces composés; les groupes alkyle de ces composés comportant de 6 à 30 atomes de carbone, notamment de 12 à 28, encore mieux de 14 à 24, voire de 16 à 22 atomes de carbone ; le groupe aryle désignant de préférence un groupe phényle ou benzyle ;
ces composés pouvant être polyoxyéthylénés ;
de préférence choisi parmi :

- les alkylsulfates notamment en C6-C24, voire en C8-C20, tel que le dodécylsulfate (mais pas utilisé à titre d'unique tensioactif mais toujours associé avec un tensioactif non ionique) ou le décylsulfate
- les alkyléthersulfates, notamment en C6-C24, voire en C12-C22, comprenant de préférence de 2 à 25 motifs oxyde d'éthylène, plus préférentiellement 2 à 10 motifs oxyde d'éthylène, tel que le lauryl éther sulfate comprenant de 2 à 25 motifs d'oxyde d'éthylène

en particulier sous forme de sels de métaux alcalins ou alcalino-terreux, d'ammonium, ou d'aminoalcool, plus particulièrement sous forme de sels de métaux alcalins tels que les sels de sodium ;
préférentiellement choisi parmi le laureth sulfate de sodium et le sulfate de sodium de décyl.

**13.** Dispersion selon l'une des revendications 1 à 8, **caractérisée en ce que** le système tensioactif comprend un tensioactif cationique.

**14.** Dispersion selon la revendication précédente, **caractérisée en ce que** le tensioactif cationique est choisi parmi les sels d'ammonium quaternaire de formule (Ia) :

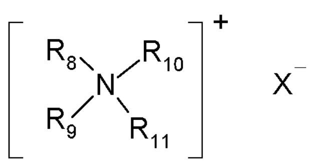

(Ia)

dans laquelle :

les groupes $R_8$ à $R_{11}$, identiques ou différents, représentent un groupe aliphatique linéaire ou ramifié, comportant de 1 à 30 atomes de carbone, ou un groupe aromatique tel que aryle ou alkylaryle, au moins l'un des groupes $R_8$ à $R_{11}$ comportant de 8 à 30 atomes de carbone, de préférence de 12 à 24 atomes de carbone; les groupes aliphatiques linéaires ou ramifiés pouvant comporter des hétéroatomes tels que notamment l'oxygène, l'azote, le soufre, ces hétéroatomes étant non adjacents, et les halogènes ; et
- $X^-$ est un anion notamment choisi dans le groupe des halogénures tels que bromures, chlorures, iodures, fluorures, phosphates, acétates, lactates, alkyl($C_1$-$C_4$)sulfates, alkyl ($C_1$-$C_4$) sulfonates ou alkyl ($C_1$-$C_4$) arylsulfonates ;

alkyle en $C_1$-$C_{30}$, alcoxy en $C_1$-$C_{30}$, polyoxyalkylène ($C_2$-$C_6$), alkylamide en $C_1$-$C_{30}$, alkyl ($C_{12}$-$C_{22}$) amidoalkyle ($C_2$-$C_6$), alkyl ($C_{12}$-$C_{22}$) acétate et hydroxyalkyle en $C_1$-$C_{30}$. ;
de préférence choisi parmi les halogénures, notamment les chlorures, de tétraalkylammonium comme les chlorures de dialkyldiméthylammonium ou d'alkyltriméthylammonium dans lesquels le groupe alkyle comporte de 12 à 22 atomes de carbone, en particulier de 14 à 20 atomes de carbone tels que les chlorures de béhényltriméthylammonium, de distéaryldiméthylammonium, de cétyltriméthylammonium (ou chlorure de cétrimonium), de benzyldiméthylstéarylammonium ; les halogénures, et notamment les chlorures, de palmitylamidopropyltriméthylammonium ou de stéaramidopropyldiméthyl-(myristyl acétate)-ammonium ;
préférentiellement choisi parmi les halogénures d'alkyltriméthylammonium dont le groupe alkyle renferme de 12 à 22 atomes de carbone, plus préférentiellement de 14 à 20 atomes de carbone et plus particulièrement les chlorures d'alkyltriméthylammonium tels que le chlorure de béhényltriméthylammonium, le chlorure de cétyltriméthylammonium.

**15.** Dispersion selon l'une quelconque des revendications précédentes, **caractérisée en ce que** le système tensioactif comprend un tensioactif non ionique.

**16.** Dispersion selon l'une quelconque des revendications précédentes, **caractérisée par le fait que** le tensioactif non ionique est choisi parmi les alcools, les alphadiols, ces composés étant polyéthoxylés et/ou polypropoxylés et/ou polyglycérolés, le nombre de groupements oxyde d'éthylène et/ou oxyde de propylène pouvant aller de 2 à 100, et le nombre de groupements glycérol pouvant aller de 2 à 30 ; ces composés comprenant au moins une chaîne grasse comportant de 8 à 30 atomes de carbone, notamment de 16 à 30 atomes de carbone ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4 ; les esters d'acides gras du sorbitane éthoxylés ayant de préférence de 2 à 40 motifs d'oxyde d'éthylène, les esters d'acides gras du saccharose, les esters d'acides gras polyoxyalkylénés, de préférence polyoxyéthylénés ayant de 2 à 150 moles d'oxyde d'éthylène ;
de préférence choisi parmi les esters d'acides gras en C8-C30 (de préférence en C12-C18) de sorbitane polyoxyéthylénés, les alcools gras en C8-C30 (de préférence en C12-18) poloxyéthylénés, les esters d'acides gras en C8-C30 (de préférence en C12-C18) polyglycérolés, les composés polyoxyéthylénés ayant de préférence 2 à 30 moles d'oxyde d'éthylène, les composés polyglycérolés ayant de préférence de 2 à 16 moles de glycérol ;
et leurs mélanges.

**17.** Dispersion selon l'une des revendications 1 à 8, **caractérisée en ce que** le système tensioactif comprend un tensioactif non ionique de HLB supérieur à 10, de préférence choisi parmi

alcool cétylique polyoxyéthyléné à 6 OE
alcool cétylique polyoxyéthyléné à 10 OE
alcool cétylique polyoxyéthyléné à 20 OE
alcool cétylique polyoxyéthyléné à 24 OE
alcool laurique polyoxyéthyléné à 7 OE
alcool laurique polyoxyéthyléné à 9 OE
alcool laurique polyoxyéthyléné à 10 OE
alcool laurique polyoxyéthyléné à 12 OE
alcool laurique polyoxyéthyléné à 23 OE
alcool stéarylique polyoxyéthyléné à 10 OE
alcool stéarylique polyoxyéthyléné à 20 OE
alcool stéarylique polyoxyéthyléné à 21 OE
monolaurate de sorbitane polyoxyéthyléné (4 OE)
monolaurate de sorbitane polyoxyéthyléné (20 OE)
monopalmitate de sorbitane polyoxyéthyléné (20 OE)
monostéarate de sorbitane polyoxyéthyléné (20 OE)
monooléate de sorbitane polyoxyéthyléné (20 OE)

**18.** Dispersion selon l'une des revendications 1 à 8, **caractérisée en ce que** le système tensioactif comprend un tensioactif de HLB inférieur ou égal à 10, de préférence choisi parmi :

alcool cétylique polyoxyéthyléné à 2 OE
alcool laurique polyoxyéthyléné à 2 OE
alcool laurique polyoxyéthyléné à 3 OE

alcool laurique polyoxyéthyléné à 4 OE
alcool stéarylique polyoxyéthyléné à 2 OE monostéarate de sorbitane polyoxyéthyléné à 4 OE polyglycéryl-4-isostéarate
préférentiellement choisi parmi polyglycéryl-4-isostéarate, alcool laurique polyoxyéthyléné à 2 OE, alcool laurique polyoxyéthyléné à 3 OE, alcool laurique polyoxyéthyléné à 4 OE.

**19.** Dispersion selon l'une des revendications 1 à 8, **caractérisée en ce que** le système tensioactif comprend un mélange de tensioactif non ionique d'alcool laurique polyoxyéthyléné 4 OE et d'alcool laurique polyoxyéthyléné 23 OE.

**20.** Dispersion selon l'une des revendications 1 à 8, **caractérisée en ce que** le système tensioactif comprend une association de tensioactif anionique et de tensioactif non ionique choisi parmi :

monopalmitate de sorbitane polyoxyéthyléné (20 OE) / sodium décyl sulfate
monopalmitate de sorbitane polyoxyéthyléné (20 OE) / N-lauroyl sarcosinate de sodium
lauryl éther sulfate / monopalmitate de sorbitane polyoxyéthyléné (20 OE)
lauryl éther sulfate / polyglycéryl-4-isostéarate lauryl éther sulfate / polyglycéryl-4-isostéarate

**21.** Dispersion selon l'une des revendications 1 à 8, **caractérisée en ce que** le système tensioactif comprend une association de tensioactif cationique et de tensioactif non ionique choisie parmi :

chlorure de cétrimonium / monopalmitate de sorbitane polyoxyéthyléné (20 OE)
chlorure de béhényl triméthylammonium / monopalmitate de sorbitane polyoxyéthyléné (20 OE)
chlorure de cétrimonium / polyglycéryl-4-isostéarate chlorure de béhényl triméthylammonium / polyglycéryl-4-isostéarate.

**22.** Dispersion selon l'une des revendications précédentes, **caractérisée en ce que** le polymère supramoléculaire est présent en une teneur allant de 2 à 50 % en poids, et de préférence allant de 5 à 40 % en poids, par rapport au poids total de la dispersion.

**23.** Dispersion selon l'une des revendications précédentes, **caractérisée en ce que** le système tensioactif est présent en une teneur allant de 0,01 à 5 % en poids, par rapport au poids total de la dispersion, notamment allant de 0,02 à 4 % en poids, de préférence allant de 0,03 à 3
% en poids, plus préférentiellement allant de 0,04 à 2 % en poids.

**24.** Dispersion selon l'une des revendications précédentes, **caractérisée en ce que** le polymère supramoléculaire et le système tensioactif sont présents selon un rapport pondéral polymère / tensioactif allant de 9 à 49, de préférence allant de 9 à 40, préférentiellement allant de 9 à 35.

**25.** Procédé de préparation d'une dispersion aqueuse de polymère telle que définie selon l'une des revendications 1 à 24, comprenant les étapes suivantes :

(i) une étape de synthèse du polymère supramoléculaire polyalcène dans un solvant organique S comme le 2-méthyl tétrahydrofurane, l'acétate d'éthyle ;
(ii) puis une étape d'addition : soit d'une solution aqueuse contenant le système de tensioactif,
soit d'une solution organique contenant le solvant organique S et le système tensioactif puis ajout d'eau ;
(iii) puis une étape de dispersion du mélange obtenu sous agitation, notamment pendant une durée allant de 1 à 60 minutes de préférence allant de 5 à 15 minutes ;
(iv) puis une étape d'évaporation du solvant organique S,

le système tensioactif étant tel que défini dans l'une des revendications 1 et 8 à 20.

**26.** Composition comprenant une dispersion aqueuse de polymère supramoléculaire polyalcène selon l'une des revendications 1 à 24.

**27.** Composition selon la revendication précédente, **caractérisée en ce qu'**elle comprend un milieu physiologiquement acceptable.

**28.** Composition selon l'une quelconque des revendications 26 et 27, dans laquelle le polymère est présent en une teneur allant de 0,1 à 50 % en poids, de préférence allant de 0,5 à 40 % en poids, préférentiellement allant de 0,5 à 30 % en poids, par rapport au poids total de la composition.

**29.** Composition selon l'une des revendications 26 à 28, **caractérisée en ce qu'**elle comprend au moins un ingrédient cosmétique choisi parmi les matières colorantes, les charges, les huiles, les cires, les pâtes, les tensioactifs (additionnels), les filtres UV, les actifs cosmétiques ; les parfums, les propulseurs, les polymères filmogènes (additionnels, notamment différents du polymère de la dispersion aqueuse), les épaississants, les conservateurs.

**30.** Procédé de traitement cosmétique des matières kératiniques comprenant l'application sur les matières kératiniques d'une composition selon l'une des revendications 26 à 29.

**31.** Procédé selon la revendication précédente, **caractérisé en ce qu'**il s'agit d'un procédé cosmétique de soin ou de maquillage des matières kératiniques.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2189151 A
- FR 2782723
- FR 2938760 A
- US 4874554 A
- US 4137180 A


### Non-patent literature cited in the description


- *J. Soc. Cosm. Chem.,* 1954, vol. 5, 249-256
- *McCutcheons Emulsifiers & Detergents,* 1998
- Kirk-Othmer's Encyclopedia of Chemical Technology. Wiley, 1979, vol. 22, 333-432